# EUROPEAN PATENT SPECIFICATION

(11) **EP 0 385 467 B1**
(45) Date of publication and mention of the grant of the patent: **15.06.1994**
(21) Application number: 90104006.3
(22) Date of filing: 01.03.1990
(51) Int. Cl.: C07K 9/00, A61K 39/39, A61K 37/02

(54) **Muramyl peptide derivatives and immunoregulating compositions containing them**
Muramylpeptid-Derivate und diese enthaltende immunregulierende Zusammensetzungen
Dérivés de muramyl peptide et compositions immunoregulantes les contenant

(30) Priority: 03.03.1989 JP 52468/89
(43) Date of publication of application: 05.09.1990
(73) Proprietor: Hasegawa, Akira, Gifu-shi Gifu 500 (JP); The Nisshin Oil Mills, Ltd., Tokyo (JP)
(72) Inventor: Hasegawa, Akira, Gifu-shi, Gifu-ken 501-31 (JP)
(74) Representative: Kraus, Walter, Dr.

(56) References cited:
- INFECTION AND IMMUNITY, vol. 53, no. 3, September 1986, pages 511-516, AmericanSociety for Microbiology, Washington, DC, US; M. TSUJIMOTO et al.: "Adjuvantactivity of 6-O-Acyl-muramyldipeptides to enhance primary cellular and humoralimmune responses in Guinea pigs: Adaptability to various vehicles andpyrogenicity"
- AGRIC. BIOL. CHEM., vol. 50, no. 8, 1986, pages 2091-2094, Tokyo, JP; A.HASEGAWA et al.: "Synthesis of N-(2-O-[2-acetamido-1-O-acyl (or 1,6-di-O-acyl)- 2,3-dideoxy-alpah-D-glucopyranose-3-yl]-D-lactoyl)-L-alanyl-D-isoglutaminemethyl esters, and their immunoadjuvant activities"
- INFECTION AND IMMUNITY, vol. 56, no. 1, January 1988, pages 149-155, AmericanSociety for Microbiology, Washington, DC, US; Y. KUMAZAWA et al.: "Importanceof fatty acid substituents of chemically synthesized lipid A-subunit analogs inthe expression of immunopharmacological activity"

## Description

The present invention relates to novel muramyl peptide derivatives. The muramyl peptide derivatives of the present invention acts on in vivo immunomechanism of human beings and livestock (in particular cells relevant to immune responses) and are useful as immunoregulating agents.

Muramyl peptides are known to possess various biological activities. That is, they possess in vitro activities such as;
(1) the action on cells related to immune responses (for example, monocytes or macrophages, B cells, T cells, natural killer (NK) cells and the like),
(2) the action on cells other than those mentioned above (for example, platelets, endothelial cells, fibroblasts and the like), and
(3) the action which activates complement systems.
   Further they show in vivo activities such as (1) immunoregulating action, and (2) enhancement of natural resistance [see Saishin Igaku, 43, No. 6, pp. 1268-1276 (1988) in Japan].

Known muramyl peptide derivatives are, for example, B30-muramyl dipeptide [Kusumoto et al; Tetrahedron letters, 49 pp. 4899-4902(1978)], muramyl dipetide-lysine [Matsumoto et al, Immunostimulants, pp. 79-97 (1987)] and those discribed in Japanese Published Unexamined Patent Application Nos. 172399/1983, 20297/1984 and 275299/1986.

Agric. Biol. Chem., 50 (8), 2091-2099, 1986 describes the synthesis of N-{2-O-[2-acetamido-1-O-acyl (or 1,6-di-O-acyl)-2,3-dideoxy-α-D-glucopyranose-3-yl]-D-lactoyl}-L-alanyl-D-isoglutamine methyl esters, and their immunoadjuvant activities.

INFECTION AND IMMUNITY, Sept. 1986, p. 511-516 describes the adjuvant activity of 6-O-acyl-muramyldipeptides to enhance primary cellular and humoral immune responses in Guinea pigs: adaptability to various vehicles and pyrogenicity.

INFECTION AND INMUNITY, Jan. 1988, p. 149-155 describes the importance of fatty acid substituents of chemically synthesized lipid A-subunit analogs in the expression of immunopharmacological activity.

However, it is still desired to develop compounds other than the known muramyl dipeptide derivatives which have more excellent activity and less toxicity.

According to the present invention, a muramyl dipeptide derivative is provided, which is represented with the following formula (I): 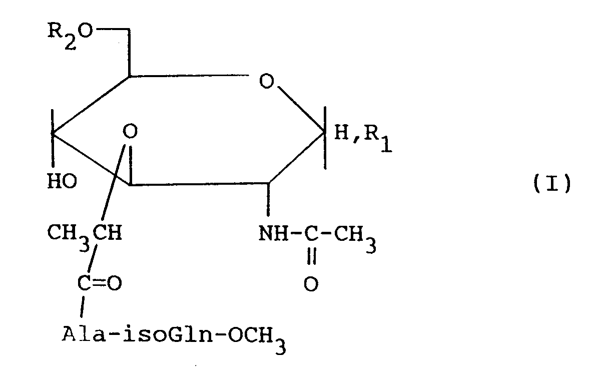
wherein "Ala" is 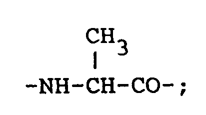
"isoGln" is 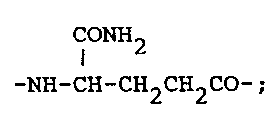
R₁ is R₃O- or R₃S- [R₃ is 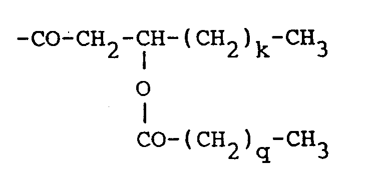
(k is an integer from 8 to 12; q is an integer from 10 to 22) or R₃ is 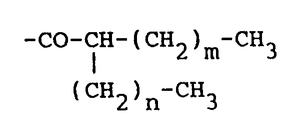
(m is an integer from 11 to 17; n is an integer from 11 to 17)]; and R₂ is a hydrogen atom or-CO-(CH₂)ₚ-CH₃ (p is an integer from 8 to 22).

The present invention also provides an immunoregulating composition comprising a compound of the formula (I) and a pharmaceutically acceptable carrier.

In the formula (I), examples of the groups R₃ in the group -OR₃ or -SR₃ include 3-dodecanoyloxy-dodecanoyl, 3-tridecanoyloxydodecanoyl, 3-tetradecanoy-loxydodecanoyl, 3-pentadecanoyloxydodecanoyl, 3-hexa-decanoyloxydodecanoyl, 3-heptadecanoyloxydodecanoyl, 3-octadecanoyloxydodecanoyl, 3-nonadecanoyloxydodecanoyl, 3-eicosanoyloxydodecanoyl, 3-docosanoyloxydodecanoyl, 3-heneicosanoyloxydodecanoyl, 3-tricosanoyloxydodecanoyl, 3-tetracosanoyloxydodecanoyl, 3-dodecanoyloxytridecanoyl, 3-tridecanoyloxytridecanoyl, 3-tetradecanoyloxytridecanoyl, 3-pentadecanoyloxytridecanoyl, 3-hexadecanoyloxytridecanoyl, 3-heptadecanoyloxytridecanoyl, 3-octadecanoyloxytridecanoyl, 3-nonadecanoyloxytridecanoyl, 3-eicosanoyloxytridecanoyl, 3-docosanoyloxytridecanoyl, 3-heneicosanoyloxytridecanoyl, 3-tricosanoyloxytridecanoyl, 3-tetracosanoyloxytridecanoyl, 3-dodecanoyloxytetradecanoyl, 3-tridecanoyloxytetradecanoyl, 3-tetradecanoyloxytetradecanoyl, 3-pentadecanoyloxytetradecanoyl, 3-hexadecanoyloxytetradecanoyl, 3-heptadecanoyloxytetradecanoyl, 3-octadecanoyloxytetradecanoyl, 3-nonadecanoyloxytetradecanoyl, 3-eicosanoyloxytetradecanoyl, 3-docosanoyloxytetradecanoyl, 3-heneicosanoyloxytetradecanoyl, 3-tricosanoyloxytetradecanoyl, 3-tetracosanoyloxytetradecanoyl, 3-dodecanoyloxypentadecanoyl, 3-tridecanoyloxypentadecanoyl, 3-tetradecanoyloxypentadecanoyl, 3-pentadecanoyloxypentadecanoyl, 3-hexadecanoyloxypentadecanoyl, 3-heptadecanoyloxypentadedanoyl, 3-octadecanoyloxypentadecanoyl, 3-nonadecanoyloxypentadecanoyl, 3-eicosanoyloxypentadecanoyl, 3-docosanoyloxypentadecanoyl, 3-heneicosanoyloxypentadecanoyl, 3-tricosnoyloxypentadecanoyl, 3-tetracosanoyloxypentadecanoyl, 3-dodecanoyloxyhexadecanoyl, 3-tridecanoyloxyhexadecanoyl, 3-tetradecanoyloxyhexadecanoyl, 3-pentadecanoyloxyhexadecanoyl, 3-hexadecanoyloxyhexadecanoyl, 3-heptadecanoyloxyhexadecanoyl, 3-octadecanoyloxyhexadecanoyl, 3-nonadecanoyloxyhexadecanoyl, 3-eicosanoyloxyhexadecanoyl, 3-docosanoyloxyhexadecanoyl, 3-heneicosanoyloxyhexadecanoyl, 3-tricosanoyloxyhexadecanoyl, 3-tetracosanoyloxyhexadecanoyl, 2-dodecyltetradecanoyl, 2-tridecyltetradecanoyl, 2-tetradecyltetradecanoyl, 2-pentadecyltetradecanoyl, 2-hexadecyltetradecanoyl, 2-heptadecyltetradecanoyl, 2-octadecyltetradecanoyl, 2-tetradecylpentadecanoyl, 2-pentadecylpentadecanoyl, 2-hexadecylpentadecanoyl, 2-heptadecylpentadecanoyl, 2-octadecylpentadecanoyl, 2-dodecylhexadecanoyl, 2-tridecylhexadecanoyl, 2-tetradecylhexadecanoyl, 2-pentadecylhexadecanoyl, 2-hexadecylhexadecanoyl, 2-heptadecylhexadecanoyl, 2-octadecylhexadecanoyl, 2-dodecylpentadecanoyl, 2-tridecylpentadecanoyl, 2-tetradecylpentadecanoyl, 2-pentadecylpentadecanoyl, 2-hexadecylpentadecanoyl, 2-heptadecylpentadecanoyl, 2-octadecylpentadecanoyl, 2-dodecylhexadecanoyl, 2-tridecylhexadecanoyl, 2-tetradecylhexadecanoyl, 2-pentadecylhexadecanoyl, 2-hexadecylhexadecanoyl, 2-heptadecylhexadecanoyl, 2-octadecylhexadecanoyl, 2-dodecylheptadecanoyl, 2-tridecylheptadecanoyl, 2-tetradecylheptadecanoyl, 2-pentadecylheptadecanoyl, 2-hexadecylheptadecanoyl, 2-octadecylheptadecanoyl, 2-dodecylocta- decanoyl, 2-tridecyloctadecanoyol, 2-tetradecylocta- decanoyl, 2-pentadecyloctadecanoyl, 2-hexadecylocta- decanoyl, 2-heptadecyloctadecanoyl, 2-octadecylocta- decanoyl, 2-dodecylnonadecanoyl, 2-tridecylnona- decanoyl, 2-tetradecylnonadecanoyl, 2-pentadecylnona- decanoyl, 2-hexadecylnonadecanoyl, 2-heptadecylnona- decanoyl, 2-octadecylnonadecanoyl, 2-dodecyleicosanoyl, 2-tridecyleicosanoyl, 2-tetradecyleicosanoyl, 2-pentadecyleicosanoyl, 2-hexadecyleicosanoyl, 2-heptadecyleicosanoyl and 2-octadecyleicosanoyl groups.

Preferred groups of R₃ are 3-tetradecanoyloxytetradecanoyl, 3-hexadecanoyloxytetradecanoyl, 3-octadecanoyloxytetradecanoyl, 3-tetracosanoyloxytetradecanoyl and 2-tetradecylhexadecanoyl groups.

Examples of R₂ include hydrogen atom, decanoyl, undecanoyl, dodecanoyl, tridecanoyl, tetradecanoyl, pentadecanoyl, hexadecanoyl, heptadecanoyl, octadecanoyl, nonadecanoyl, eicosanoyl, docosanoyl, heneicosanoyl, tricosanoyl and tetracosanoyl groups.

R₂ is preferably hydrogen atom or tetradecanoyl group.

Preferably, "Ala" is an L-alanine residue, and "isoGln" is a residue derivated from D-isoglutamine.

The compounds of the formula (I) of the present invention are basically muramyl dipeptide derivatives, in which the muramyl dipeptide moiety has preferably the same steric configuration as that of the muramyl dipeptide moiety in natural muramyl dipeptides. Namely, the moieties of muraminic acid and dipeptide in the present muramyl dipeptides have D-steric configuration and L-alanine-D-isoglutamine configuration, respectively. However, the muramyl dipeptides of the present invention may be those having other possible steric configurations.

The group -OR₃ or -SR₃ in the definition of the formula (I) preferably combines with the saccharide moiety in the form of α-bond and β-bond, respectively.

The acyloxyacyl group in R₃ has an asymmetric carbon atom and may be in the form of D- or L-isomer, or racemic mixture.

Interesting compounds belonging to the formula (I) in the present invention include:
N-[2-0-{2-Acetamido-2,3-dideoxy-1-0-(2-tetradecylhexadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0(2-tetradecylhexadecanoyl)-α-D-glucopyranos-3-yl}-D-lactoyl]-L-alanyl-D-isoglutamine methylester
N-[2-0-{2-Acetamido-2,3-dideoxy-6-0-tetradecanoyl-1-0-(2-tetradecylhexadecanoyl)-α-D-glucopyranos-3-yl}-D-lactoyl]-L-alanyl-D-isoglutamine methylester
N-[2-0-{2-Acetamido-2,3-dideoxy-6-0-octadecanoyl-1-0-(2-tetradecylhexadecanoyl)-α-D-glucopyranos-3-yl}-D-lactoyl]-L-alanyl-D-isoglutamine methylester
N-[2-0-{2-Acetamido-2,3-dideoxy-1-S-(2-tetradecylhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-(2-tetradecylhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-tetradecanoyl-1-S-(2-tetradecylhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-octadecanoyl-1-S-(2-tetradecylhexadecanoyl)-1-thio-β-D-glucopyranos-3-yl}-D-lactoyl]-L-alanyl-D-isoglutamine methylester
N-[2-0-{2-Acetamido-2,3-dideoxy-1-0-((3R)-3-tetradecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-tetradecanolyloxytetradecanoyl)-α-D-glucopyranos-3-yl}-D-lactoyl]-L-alanyl-D-isoglutamine methylester
N-[2-0-{2-Acetamido-2,3-dideoxy-6-0-tetradecanoyl-1-0-((3R)-3-tetradecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-octadecanoyl-1-0-((3R)-3-tetradecanoyloxytetradecanoyl)-α-D-glucopyranos-3-yl}-D-lactoyl]-L-alanyl-D-isoglutamine methylester
N-[2-0-{2-Acetamido-2,3-dideoxy-1-S-((3R)-3-tetradecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-tetradecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-tetradecanoyl-1-S-((3R)-3-tetradecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-octadecanoyl-1-S-((3R)-3-tetradecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-1-0-((3R)-3-hexadecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-hexadecanoyloxytetradecanoyl)-α-D-glucopyranos-3-yl}-D-lactoyl]-L-alanyl-D-isoglutamine methylester
N-[2-0-{2-Acetamido-2,3-dideoxy-1-0-((3R)-3-hexadecanoyloxytetradecanoyl-6-0-tetradecanoyl-α-D-glucopyranos-3-yl}-D-lactoyl]-L-alanyl-D-isoglutamine methylester
N-[2-0-{2-Acetamido-2,3-dideoxy-1-0-((3R)-3-hexadecanoyloxytetradecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-hexadecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-hexadecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-hexadecanoyloxytetradecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-hexadecanoyloxytetradecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-octadecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-hexadecanoyloxytetradecanoyl)-α-D-glucopyranos-3-yl}-D-lactoyl]-L-alanyl-D-isoglutamine methylester
N-[2-0-{2-Acetamido-2,3-dideoxy-1-0-((3R)-3-octadecanoyloxytetradecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-octadecanoyl-1-0-((3R)-3-octadecanoyloxytetradecanoyl)-α-D-gluco pyranos-3-yl}-D-lactoyl]-L-alanyl-D-isoglutamine methylester
N-[2-0-{2-Acetamido-2,3-dideoxy-1-S-((3R)-3-octadecanoyloxytetradecanoyl)-1-thio-β-D-glucopyranos-3-yl}-D-lactoyl]-L-alanyl-D-isoglutmine methylester
N-[2-0-{2-Acetamido-2,3-dideoxy-6-0-decanoyl-1-S-((3R)-3-octadecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-octadecanoyloxytetradecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-octadecanoyl-1-S-((3R)-3-octadecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-1-0-((3R)-3-tetracosanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-tetracosanoyloxytetradecanoyl)-α-D-glucopyranos-3-yl}-D-lactoyl]-L-alanyl-D-isoglutamine methylester
N-[2-0-{2-Acetamido-2,3-dideoxy-1-0-((3R)-3-tetracosanoyloxytetradecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-octadecanoyl-1-0-((3R)-3-tetracosanoyloxytetradecanoyl)-α-D-glucopyranos-3-yl}-D-lactoyl]-L-alanyl-D-isoglutamine methylester
N-[2-0-{2-Acetamido-2,3-dideoxy-1-S-((3R)-3-tetracosanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-tetracosanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-tetracosanoyloxytetradecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-octadecanoyl-1-S-((3R)-3-tetracosanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-0-(2-tetradecylhexadecanoyl)-α-D-glucopyranos-3-yl}-d-lactoyl]-L-alanyl-D-isoglutamine methylester
N-[2-0-{2-Acetamido-2,3-dideoxy-6-0-hexadecanoyl-1-0-(2-tetradecylhexadecanoyl)-α-D-glucpyranos-3-yl}-D-lactoyl]-L-alanyl-D-isoglutamine methylester
N-[2-0-(2-Acetamido-2,3-dideoxy-6-0-dodecanoyl-1-S-(2-tetradecylhexadecanoyl)-1-thio-β-D-glucopyranos-3-yl}-D-lactoyl]-L-alanyl-D-isoglutamine methyester
N-[2-0-(2-Acetamido-2,3-dideoxy-6-0-hyxadecanoyl-1-S-(2-tetradecylhexadecanoyl)-1-thio-β-D-glucopyranos-3-yl}-D-lactoyl]-L-alanyl-D-isoglutamine methyester
N-[2-0-{2-Acetamido-2,3-dideoxy-1-0-((3R)-3-dodecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-dodecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-dodecanoyloxytetradecanoyl)-α-D-glucopyranos-3-yl}-D-lactoyl]-L-alanyl-D-isoglutamine methylester
N-[2-0-{2-Acetamido-2,3-dideoxy-1-0-((3R)-3-dodecanoyloxytetradecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-((3R)-3-dodecanoyloxytetradecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-((3R)-3-dodecanoyloxytetradecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-dodecanoyloxytetradecnaoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-dodecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-dodecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-dodecanoyloxytetradecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-dodecanoyloxytetradecanoyl)-6-0-hexadecanoyl-1-thio-β- D-glucopyranos-3-yl}-D-lactoyl]-L-alanyl-D-isoglutamine methylester
N-[2-0-{2-Acetamido-2,3-dideoxy-1-S-((3R)-3-dodecanoyloxytetradecanoyl-6-0-octadecanoyl-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-0-dodecanoyl-1-0-((3R)-3-tetradecanoyloxytetradecanoyl-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-tetradecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-tetradecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-tetradecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-hexadecanoyloxytetradecanoyl)-α-D-gluco pyranos-3-yl}-D-lactoyl]-L-alanyl-D-isoglutamine methylester
N-[2-0-{2-Acetamido-2,3-dideoxy-6-0-hexadecanoyl-1-0-((3R)-3-hexadecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-hexadecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-hexadecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-hexadecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-octadecanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-octadecanoyloxytetradecanoyl)-1-thio-β-D- glucopyranos-3-yl}-D-lactoyl]-L-alanyl-D-isoglutamine methylester
N-[2-0-(2-Acetamido-2,3-dideoxy-6-0-hexadecanoyl-1-S-((3R)-3-octadecanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-1-0-((3R)-3-eicosanoyloxytetradecanoyl)-α-D-glucopyranos-3-yl}-D-lactoyl]- L-alanyl-D-isoglutamine methylester
N-[2-0-{2-Acetamido-2,3-dideoxy-6-0-decanoyl-1-0-((3R)-3-eicosanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-eicosanoyloxytetradecanoyl)-α-D-glucopyranos-3-yl}-D-lactoyl]-L-alanyl-D-isoglutamine methylester
N-[2-0-{2-Acetamido-2,3-dideoxy-1-0-((3R)-3-eeicosanoyloxytetradecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-((3R)-3-eicosanoyloxytetradecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-((3R)-3-eicosanoyloxytetrdecanoyl)-6-0-octadecanoyl-α-D-gluco pyranos-3-yl}-D-lactoyl]-L-alanyl-D-isoglutamine methylester
N-[2-0-{2-Acetamido-2,3-dideoxy-1-S-((3R)-3-eicosanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-eicosanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-eicosanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-eicosanoyloxytetradecanoyl)-6-0-tetradecanoyl1-thio-β-D-glucopyranos-3-yl}-D-lactoyl]-L-alanyl-D-isoglutamine methylester
N-[2-0-{2-Acetamido-2,3-dideoxy-1-S-((3R)-3-eicosanoyloxytetradecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-eicosanoyloxytetradecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-docosanoyloxytetradecanoyl)-α-D-glucopyranos-3-yl}-D-1-actoyl]-L-alanyl-D-isoglutamine methylester
N-[2-0-{2-Acetamido-2,3-dideoxy-6-0-decanoyl-1-0-((3R)-3-docosanoyloxytetradecanoyl)-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxytetradecanoyl)-6-0-decanoyl-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxytetradecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxytetradecanoyl-6-0-hexadecanoyl-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxytetradecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-docosanoyloxytetradecanoyl)-1-thio-β-D-glucopyranos- 3-yl}-D-lactoyl]-L-alanyl-D-isoglutamine methylester
N-[2-0-{2-Acetamido-2,3-dideoxy-1-S-((3R)-3-docosanoyloxytetradecanoyl)-1-thio-β-D-glucopyranos-3-yl}-D-lactoyl]-L-alanyl-D-isoglutamlne methylester
N-[2-0-{2-Acetamido-2,3-dideoxy-1-S-((3R)-3-docosanoyloxytetradecanoyl)-6-0-dodecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-docosanoyloxytetradecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-docosanoyloxytetradecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-docosanoyloxytetradecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-tetracosanoyloxytetradecaoyl)-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-tetracosanoyloxytetradecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-tetracosanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-tetracosanoyloxytetradecanoyl)-1-thio-β-D-glucopyranos-3-yl}-D-lactoyl]-L-alanyl-D-isoglutamine methylester
N-[2-0-{2-Acetamido-2,3-dideoxy-1-0-(2-dodecyltetradecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-(2-dodecyltetradecanoyl)-α-D-glucopyranos-3-yl}-D-lactoyl]-L-alanyl-D-isoglutamine methylester
N-[2-0-{2-Acetamldo-2,3-dideoxy-6-0-dodecanoyl-1-0-(2-dodecyltetradecanoyl)-α-D-glucopyranos-3-yl}-D-lactoyl]-L-alanyl-D-isoglutamine methylester
N-[2-0-{2-Acetamido-2,3-dideoxy-1-0-(2-dodecyltetradecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-(2-dodecyltetradecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-(2-dodecyltetradecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-(2-dodecyltetradecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-(2-dodecyltetradecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-(2-dodecyltetradecanoyl)-1-thio-β-D-glucopyranos-3-yl}-D-lactoyl]-L-alanyl-D-isoglutamine methylester
N-[2-0-{2-Acetamido-2,3-dideoxy-1-S-(2-dodecyltetradecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-(2-dodecyltetradecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-(2-dodecyltetradecanoyl)-6-0-octadecanoyl-1-thio-β-D-gluco pyranos-3-yl)-D-lactoyl]-L-alanyl-D-isoglutamine methylester
N-[2-0-{2-Acetamido-2,3-dideoxy-1-0-(2-hexadecyloctadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-(2-hexadecyloctadecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-(2-hexadecyloctadecanoyl)-α-D-glucopyranos-3-yl}-D-lactoyl]-L-alanyl-D-isoglutamine methylester
N-[2-0-{2-Acetamido-2,3-dideoxy-1-0-(2-hexadecyloctadecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-hexadecanoyl-1-0-(2-hexadecyloctadecanoyl)-α-D-glucopyranos-3-yl}-D-lactoyl]-L-alanyl-D-isoglutamine methylester
N-[2-0-{2-Acetamido-2,3-dideoxy-1-0-(2-hexadecyloctadecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-(2-hexadecyloctadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-(2-hexadecyloctadecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-(2-hexadecyloctadecanoyl)-1-thio-β-D-glucopyranos-3-yl}-D-lactoyl]-L-alanyl-D-isoglutamine methylester
N-[2-0-{2-Acetamido-2,3-dideoxy-1-S-(2-hexadecyloctadecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-(2-hexadecyloctadecanoyl)-1-thio-β-D-glucopyranos-3-yl}-D-lactoyl]-L-alanyl-D-isoglutamine methylester
N-[2-0-{2-Acetamido-2,3-dideoxy-1-S-(2-hexadecyloctadecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-(2-octadecyleicosanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-(2-octadecyleisocanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-(2-octadecyleicosanoyl)-α-D-glucopyranos-3-yl}-D-lactoyl]-L-alanyl-D-isoglutamine methylester
N-[2-0-{2-Acetamido-2,3-dideoxy-1-0-(2-octadecyleicosanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-hexadecanoyl-1-0-(2-octadecyleicosanoyl)-α-D-glucopyranos-3-yl}-D-lactoyl]-L-alanyl-D-isoglutamine methylester
N-[2-0-{2-Acetamido-2,3-dideoxy-6-0-octadecanoyl-1-0-(2-octadecyleicosanoyl)-α-D-glucopyranos-3-yl}-D-lactoyl]-L-alanyl-D-isoglutamlne methylester
N-[2-0-{2-Acetamido-2,3-dideoxy-1-S-(2-octadecyleicosanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-(2-octadecyleicosanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-(2-octadecyleicosanoyl)-1-thio-β-D-glucopyranos-3-yl}-D-lactoyl]-L-alanyl-D-isoglutamine methylester
N-[2-0-{2-Acetamido-2,3-dideoxy-1-S-(2-octadecyleicosanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-(2-octadecyleicosanoyl)-1-thio-β-D-glucopyranos-3-yl}-D-lactoyl]-L-alanyl-D-isoglutamine methylester
N-[2-0-{2-Acetamido-2,3-dideoxy-6-0-octadecanoyl-1-S-(2-octadecyleicosanoyl)-1-thio-β-D-glucopyranos-3-yl}-D-lactoyl]-L-alanyl-D-isoglutamine methylester
N-[2-0-{2-Acetamido-2,3-dideoxy-1-0-(2-dodecylhexadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-(2-dodecylhexadecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-(2-dodecylhexadecanoyl)-α-D-glucopyranos-3-yl}-D-lactoyl]-L-alanyl-D-isoglutamine methylester
N-[2-0-{2-Acetamido-2,3-dideoxy-1-0-(2-dodecylhexadecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-(2-dodecylhexadecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-(2-dodecylhexadecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-(2-dodecylhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-(2-dodecylhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-(2-dodecylhexadecanoyl)-1-thio-β-D-glucopyranos-3-yl}-D-lactoyl]-L-alanyl-D-isoglutamine methylester
N-[2-0-{2-Acetamido-2,3-dideoxy-1-S-(2-dodecylhexadecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-(2-dodecylhexadecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-(2-dodecylhexadecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-(2-dodecyloctadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-(2-dodecyloctadecanoyl)-α-D-glucopyranos-3-yl}-D-lactoyl]-L- alanyl-D-isoglutamine methylester
N-[2-0-{2-Acetamido-2,3-dideoxy-6-0-dodecanoyl-1-0-(2-dodecyloctadecanoyl)-α-D-glucopyranos-3-yl}-D-lactoyl]-L-alanyl-D-isoglutamine methylester
N-[2-0-{2-Acetamido-2,3-dideoxy-1-0-(2-dodecyloctadecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-(2-dodecyloctadecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-(2-dodecyloctadecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-(2-dodecyloctadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-(2-dodecyloctadecanoyl)-1-thio-β-D-glucopyranos-3-yl}-D-lactoyl]-L-alanyl-D-isoglutamine methylester
N-[2-0-{2-Acetamido-2,3-dideoxy-6-0-docanoyl-1-S-(2-dodecyloctadecanoyl)-1-thio-β-D-glucopyranos-3-yl}-D-lactoyl]-L-alanyl-D-isoglutamine methylester
N-[2-0-{2-Acetamido-2,3-dideoxy-1-S-(2-dodecyloctadecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-(2-dodecyloctadecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-(2-dodecyloctadecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-(2-dodecyleicosanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-(2-dodecyleicosanoyl)-α-D-glucopyranos-3-yl}-D-lactoyl]-L-alanyl- D-isoglutamine methylester
N-[2-0-{2-Acetamido-2,3-dideoxy-6-0-dodecanoyl-1-0-(2-dodecyleicosanoyl)-α-D-glucopyranos-3-yl}-D-lactoyl]-L-alanyl-D-isoglutamine methylester
N-[2-0-{2-Acetamido-2,3-dideoxy-1-0-(2-dodecyleicosanoyl)-6-0-tetradecnaoyl-α-D-glucopyranos-3-yl}-D-lactoyl]-L-alanyl-D-isoglutamine methylester
N-[2-0-{2-Acetamido-2,3-dideoxy-1-0-(2-dodecyleicosanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-(2-dodecyleicosanoyl)-6-0-octadecanoyl)-α-D-glucopyranos-3-yl}-D-lactoyl]-L-alanyl-D-isoglutamine methylester
N-[2-0-{2-Acetamido-2,3-dideoxy-1-S-(2-dodecyleicosanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-(2-dodecyleicosanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-(2-dodecyleicosanoyl)-1-thio-β-D-glucopyranos-3-yl}-D-lactoyl]-L-alanyl-D-isoglutamine methylester
N-[2-0-{2-Acetamido-2,3-dideoxy-1-S-(2-dodecyleicosanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-(2-dodecyleicosanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-(2-dodecyleicosanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-dodecanoyloxydodecanoyl)-α-D-glucopyranos-3-yl}-D-lactoyl]-L-alanyl-D-isoglutamine methylester
N-[2-0-{2-Acetamido-2,3-dideoxy-6-0-docanoyl-1-0-((3R)-3-dodecanoyloxydodecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-dodecanoyloxydodecanoyl)-α-D-glucopyranos-3-yl}-D-lactoyl]-L-alanyl-D-isoglutamine methylester
N-[2-0-{2-Acetamido-2,3-dideoxy-1-0-((3R)-3-dodecanoyloxydodecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-((3R)-3-dodecanoyloxydodecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-((3R)-3-dodecanoyloxydodecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-dodecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-docanoyl-1-S-((3R)-3-dodecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-dodecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-dodecanoyloxydodecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-dodecanoyloxydodecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-dodecanoyloxydodecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-tetradecanoyloxydodecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-tetadecanoyloxydodecanoyl)-α-D-glucopyrano-s-3- yl}-D-lactoyl]-L-alanyl-D-isoglutamine methylester
N-[2-0-{2-Acetamido-2,3-dideoxy-6-0-dodecanoyl-1-0-((3R)-3-tetradecanoyloxydodecanoyl)-α-D-glucopyranos-3-yl}-D-lactoyl]-L-alanyl-D-isoglutamine methylester
N-[2-0-{2-Acetamido-2,3-dideoxy-6-0-tetradecanoyl-1-0-((3R)-3-ttradecanoyloxydodecanoyl)-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-tetradecanoyloxydodecanoyl)-α-D-glucopyranos-3-yl}-D-lactoyl]-L-alanyl-D-isoglutamine methylester
N-[2-0-{2-Acetamido-2,3-dideoxy-6-0-octadecanoyl-1-0-((3R)-3-tetradecanoyloxydodecanoyl)-α-D-glucopyranos-3-yl}-D-lactoyl]-L-alanyl-D-isoglutamine methylester
N-[2-0-{2-Acetamido-2,3-dideoxy-1-S-((3R)-3-tetradecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-tetradecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-tetradecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-tetradecanoyl-1-S-((3R)-3-tetradecanoyloxydodecanoyl)-1-thio-β-D- glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-tetradecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-octadecanoyl-1-S-((3R)-3-tetradecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-1-0-((3R)-3-hexadecanoyloxydodecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-hexadecanoyloxydodecanoyl-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-hexadecanoyloxydodecanoyl)-α-D-glucopyranos-3-yl}-D-lactoyl]-L-alanyl-D-isoglutamine methylester
N-[2-0-{2-Acetamido-2,3-dideoxy-1-0-((3R)-3-hexadecanoyloxydodecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-hexadecanoyl1-0-((3R)-3-hexadecanoyloxydodecanoyl)-α-D-glucopyranos-3-yl}-D-lactoyl]-L-alanyl-D-isoglutamine methylester
N-[2-0-{2-Acetamido-2,3-dideoxy-1-0-((3R)-3-hexadecanoyloxydodecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-hexadecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-hexadecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-hexadecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-hexadecanoyloxydodecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-hexadecanoyloxydodecanoyl)-1-thio-β-D- glucopyranos-3-yl}-D-lactoyl]-L-alanyl-D-isoglutamine methylester
N-[2-0-{2-Acetamido-2,3-dideoxy-1-S-((3R)-3-hexadecanoyloxydodecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-octadecanoyloxydodecanoyl)-α-D-glucopyranos-3-yl}-D-lactoyl]-L-alanyl-D-isoglutamine methylester
N-[2-0-{2-Acetamido 2,3-dideoxy-6-0-decanoyl-1-0-((3R)-3-octadecanoyloxydodecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-octadecanoyloxydodecanoyl)-α-D-glucopyranos-3-yl}-D-lactoyl]-L-alanyl-D-isoglutamine methylester
N-[2-0-{2-Acetamido-2,3-dideoxy-1-0-((3R)-3-octadecanoyloxydodecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-octadecanoyloxydodecanoyl)-α-D-glucopyranos-3-yl}-D-lactoyl]-L-alanyl-D-isoglutamine methylester
N-[2-0-{2-Acetamido-2,3-dideoxy-6-0-octadecanoyl-1-0-((3R)-3-octadecanoyloxydodecanoyl)-α-D-gluco pyranos-3-yl}-D-lactoyl]-L-alanyl-D-isoglutamine methylester
N-[2-0-{2-Acetamido-2,3-dideoxy-1-S-((3R)-3-octadecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-octadecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-octadecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-octadecanoyloxydodecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-octadecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-octadecanoyl-1-S-((3R)-3-octadecanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-1-0-((3R)-3-eicosanoyloxydodecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-eicosanoyloxydodecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-eicosanoyloxydodecanoyl)-α-D-glucopyranos-3-yl}-D-lactoyl]-L-alanyl-D-isoglutamine methylester
N-[2-0-{2-Acetamido-2,3-dideoxy-1-0-((3R)-3-eicosanoyloxydodecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-((3R)-3-eicosanoyloxydodecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-((3R)-3-eicosanoyloxydodecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-eicosanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-eicosanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-eicosanoyloxydodecanoyl)-1-thio-β-D-glucopyranos-3-yl)-D-lactoyl]-L-alanyl-D-isoglutamine methylester
N-[2-0-{2-Acetamido-2,3-dideoxy-1-S-((3R)-3-eicosanoyloxydodecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-eicosanoyloxydodecanoly)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-eicosanoyloxydodecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-docosanoyloxydodecanol)-α-D-glucopyranos-3-yl}-D-lactoyl]-L-alanyl-D-isoglutamine methylester
N-[2-0-{2-Acetamido-2,3-dideoxy--6-0-decanoyl-1-0-((3R)-3-docosanoyloxydodecanoyl)-α-D-gluco pyranos-3-yl}-D-lactoyl]-L-alanyl-D-isoglutamine methylester
N-[2-0-{2-Acetamido-2,3-dideoxy-1-0-((3R)-3-docosanoyloxydodecanoyl)-6-0-dodecanoyl-α-D-glucopyranos-3-yl}-D-lactoyl]-L-alanyl-D-isoglutamine-methylester
N-[2-0-{2-Acetamido-2,3-dideoxy-1-0-((3R)-3-docosanoyloxydodecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxydodecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxydodecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-docosanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-docosanoyloxydodecanoyl)-1-thio-β-D-gluco pyranos-3-yl}-D-lactoyl]-L-alanyl-D-isoglutamine methylester
N-[2-0-{2-Acetamido-2,3-dideoxy-1-S-((3R)-3-docosanoyloxydodecanoyl)-6-0-dodecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-docosanoyloxydodecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-docosanoyloxydodecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-docosanoyloxydodecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-tetracosanoyloxydodecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-tetracosanoyloxydodecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-tetracosanoyloxydodecanoyl)-α-D-glucopyranos-3-yl}-D-lactoyl]-L-alanyl-D-isoglutamine methylester
N-[2-0-{2-Acetamido-2,3-dideoxy-1-0-((3R)-3-tetracosanoyloxydodecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-tetracosanoyloxydodecanoyl)-α-D-glucopyranos-3-yl}-D-lactoyl]-L-alanyl-D-isoglutamine methylester
N-[2-0-{2-Acetamido-2,3-dideoxy-6-0-octadecanoyl-1-0-((3R)-3-tetracosanoyloxydodecanoyl)-α-D-glucopyranos-3-yl}-D-lactoyl]-L-alanyl-D-isoglutamine methylester
N-[2-0-{2-Acetamido-2,3-dideoxy-1-S-((3R)-3-tetracosanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-tetracosanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-tetracosanoyloxydodecanoyl)-1-thio-β-D- glucopyranos-3-yl}-D-lactoyl]-L-alanyl-D-isoglutamine methylester
N-[2-0-{2-Acetamido-2,3-dideoxy-1-S-((3R)-3-tetracosanoyloxydodecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-tetracosanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-6-0-octadecanoyl-1-S-((3R)-3-tetracosanoyloxydodecanoyl)-1-thio-β-D-glucopyranos-3-yl}-D-lactoyl]-L-alanyl-D-isoglutamine methylester
N-[2-0-{2-Acetamido-2,3-dideoxy-1-0-((3R)-3-dodecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-dodecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-dodecanoyloxyhexadecanoyl)-α-D-glucopyranos-3-yl}-D-lactoyl]-L-alanyl-D-isoglutamine methylester
N-[2-0-{2-Acetamido-2,3-dideoxy-1-0-((3R)-3-dodecanoyloxyhexadecanoyl)-6-0-tetradecanoyl-α-D-gluco pyranos-3-yl}-D-lactoyl]-L-alanyl-D-isoglutamine methylester
N-[2-0-{2-Acetamido-2,3-dideoxy-1-0-((3R)-3-dodecanoyloxyhexadecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-((3R)-3-dodecanoyloxyhexadecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-dodecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-dodecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-dodecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-dodecanoyloxyhexadecanoyl)-6-0-tetradecanoyl-1-thio-β- D-glucopyranos-3-yl}-D-lactoyl]-L-alanyl-D-isoglutamine methylester
N-[2-0-{2-Acetamido-2,3-dideoxy-1-S-((3R)-3-dodecanoyloxyhexadecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-dodecanoyloxyhexadecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-tetradecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-tetradecanoyloxyhexadecanoyl)-α-D-glucopyranos-3-yl}-D-lactoyl]-L-alanyl-D-lsoglutamine methylester
N-[2-0-{2-Acetamido-2,3-dideoxy-6-0-dodecanoyl-1-0-((3R)-3-tetradecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-tetradecanoyl-1-0-((3R)-3-tetradecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-tetradecanoyloxyhexadecanoyl)-α-D-gluco pyranos-3-yl}-D-lactoyl]-L-alanyl-D-isoglutamine methylester
N-[2-0-{2-Acetamido-2,3-dideoxy-6-0-octadecanoyl-1-0-((3R)-3-tetradecanoyloxyhexadecanoyl)-α-D-glucopyranos-3-yl}-D-lactoyl]-L-alanyl-D-isoglutamine methylester
N-[2-0-{2-Acetamido-2,3-dideoxy-1-S-((3R)-3-tetradecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-tetradecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-tetradecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-tetradecanoyl-1-S-((3R)-tetradecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-tetradecanoyloxyhexadecanoyl)-1-thio-β-D- glucopyranos-3-yl}-D-lactoyl]-L-alanyl-D-isoglutamine methylester
N-[2-0-{2-Acetamido-2,3-dideoxy-6-0-octadecanoyl-1-S-((3R)-3-tetradecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-1-0-((3R)-3-hexadecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-hexadecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-hexadecanoyloxyhexadecanoyl)-α-D-glucopyranos-3-yl}-D-lactoyl]-L-alanyl-D-isoglutamine methylester
N-[2-0-{2-Acetamido-2,3-dideoxy-1-0-((3R)-3-hexadecanoyloxyhexadecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-hexadecanoyloxyhexadecanoyl)-α-D-gluco pyranos-3-yl}-D-lactoyl]-L-alanyl-D-isoglutamine methylester
N-[2-0-{2-Acetamido-2,3-dideoxy-1-0-((3R)-3-hexadecanoyloxyhexadecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-hexadecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-hexadecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-hexadecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-hexadecanoyloxyhexadecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-hexadecanoyloxyhexadecanoyl)-1-thio-β-D- glucopyranos-3-yl}-D-lactoyl]-L-alanyl-D-isoglutamine methylester
N-[2-0-{2-Acetamido-2,3-dideoxy-1-S-((3R)-3-hexadecanoyloxyhexadecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-octadecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-octadecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-octadecanoyloxyhexadecanoyl)-α-D-glucopyranos-3-yl}-D-lactoyl]-L-alanyl-D-isoglutamine methylester
N-[2-0-{2-Acetamido-2,3-dideoxy-1-0-((3R)-3-octadecanoyloxyhexadecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-octadecanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-octadecanoyl-1-0-((3R)-3-octadecanoyloxyhexadecanoyl)-α-D-glucopyranos-3-yl}-D-lactoyl]-L-alanyl-D-isoglutamine methylester
N-[2-0-{2-Acetamido-2,3-dideoxy-1-S-((3R)-3-octadecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-octadecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-octadecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-octadecanoyloxyhexadecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-octadecanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-octadecanoyl-1-S-((3R)-3-octadecanoyloxyhexadecanoyl)-1-thio-β-D- glucopyranos-3-yl}-D-lactoyl]-L-alanyl-D-isoglutamine methylester
N-[2-0-{2-Acetamido-2,3-dideoxy-1-0-((3R)-3-eicosanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-eicosanoyloxyhexadecanoyl-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-eicosanoyloxyhexadecanoyl)-α-D-glucopyranos-3-yl}-D-lactoyl]-L-alanyl-D-isoglutamine methylester
N-[2-0-{2-Acetamido-2,3-dideoxy-1-0-((3R)-3-eicosanoyloxyhexadecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-((3R)-3-eicosanoyloxyhexadecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-((3R)-3-eicosanoyloxyhexadecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-eicosanoyloxyhexadecanoyl)-1-thio-β-D-glucopyranos-3-yl}-D-lactoyl]-L-alanyl-D-isoglutamlne methylester
N-[2-0-{2-Acetamido-2,3-dideoxy-6-0-decanoyl-1-S-((3R)-3-eicosanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-dodecanoyl-1-S-((3R)-3-eicosanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-eicosanoyloxyhexadecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-eicosanoyloxyhexadecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-eicosanoyloxyhexadecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-docosanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-docosanoyloxyhexadecanoyl)-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxyhexadecanoyl)-6-0-dodecanoyl-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxyhexadecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxyhexadecanoyl)-6-0-hexadecanoyl-α-D-glucopyranos-3-yl}-D-lactoyl]-L-alanyl-D-isoglutamine methylester
N-[2-0-{2-Acetamido-2,3-dideoxy-1-0-((3R)-3-docosanoyloxyhexadecanoyl)-6-0-octadecanoyl-α-D-glucopyranos-3-yl}-D-lactoyl]-L-alanyl-D-isoglutamine methylester
N-[2-0-{2-Acetamido-2,3-dideoxy-1-S-((3R)-3-docosanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-docosanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-docosanoyloxyhexadecanoyl)-6-0-dodecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-docosanoyloxyhexadecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-docosanoyloxyhexadecanoyl)-6-0-hexadecanoyl-1-thio-β-D-glucopyranos-3-yl}-D-lactoyl]-L-alanyl-D-isoglutamine methylester
N-[2-0-{2-Acetamido-2,3-dideoxy-1-S-((3R)-3-docosanoyloxyhexadecanoyl)-6-0-octadecanoyl-1-thio-β-D-glucopyranos-3-yl}-D-lactoyl]-L-alanyl-D-isoglutamine methylester
N-[2-0-{2-Acetamido-2,3-dideoxy-1-0-((3R)-3-tetracosanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-decanoyl-1-0-((3R)-3-tetracosanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-dodecanoyl-1-0-((3R)-3-tetracosanoyloxyhexadecanoyl)-α-D-glucopyranos-3-yl}-D-lactoyl]-L-alanyl-D-isoglutamine methylester
N-[2-0-{2-Acetamido-2,3-dideoxy-1-0-((3R)-3-tetracosanoyloxyhexadecanoyl)-6-0-tetradecanoyl-α-D-glucopyranos-3-yl}-D-lactoyl]-L-alanyl-D-isoglutamine methylester
N-[2-0-{2-Acetamido-2,3-dideoxy-6-0-hexadecanoyl-1-0-((3R)-3-tetracosanoyloxyhexadecanoyl)-α-D-glucopyranos-3-yl}-D-lactoyl]-L-alanyl-D-isoglutamine methylester
N-[2-0-{2-Acetamido-2,3-dideoxy-6-0-octadecanoyl-1-0-((3R)-3-tetracosanoyloxyhexadecanoyl)-α-D-glucopyranos-3-yl}-D-lactoyl]-L-alanyl-D-isoglutamine methylester
N-[2-0-{2-Acetamido-2,3-dideoxy-1-S-((3R)-3-tetracosanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-6-0-decanoyl-1-S-((3R)-3-tetracosanoyloxyhexadecanoyl)-1-thio-β-D-gluco pyranos-3-yl}-D-lactoyl]-L-alanyl-D-isoglutamine methylester
N-[2-0-{2-Acetamido-2,3-dideoxy-6-0-dodecanoyl-1-S-((3R)-3-tetracosanoyloxyhexadecanoyl)-1-thio-β-D-glucopyranos-3-yl}-D-lactoyl]-L-alanyl-D-isoglutamine methylester
N-[2-0-{2-Acetamido-2,3-dideoxy-1-S-((3R)-3-tetracosanoyloxyhexadecanoyl)-6-0-tetradecanoyl-1-thio-β-D-glucopyranos-3-yl}-D-lactoyl]-L-alanyl-D-isoglutamine methylester
N-[2-0-{2-Acetamido-2,3-dideoxy-6-0-hexadecanoyl-1-S-((3R)-3-tetracosanoyloxyhexadecanoyl)-1-thio-β-D-glucopyranos-3-yl}-D-lactoyl]-L-alanyl-D-isoglutamine methylester and
N-[2-0-{2-Acetamido-2,3-dideoxy-6-0-octadecanoyl-1-S-((3R)-3-tetracosanoyloxyhexadecanoyl)-1-thio-β-D-glucopyranos-3-yl}-D-lactoyl]-L-alanyl-D-isoglutamine methylester.

The compounds of the present invention can be basically prepared by the following process. 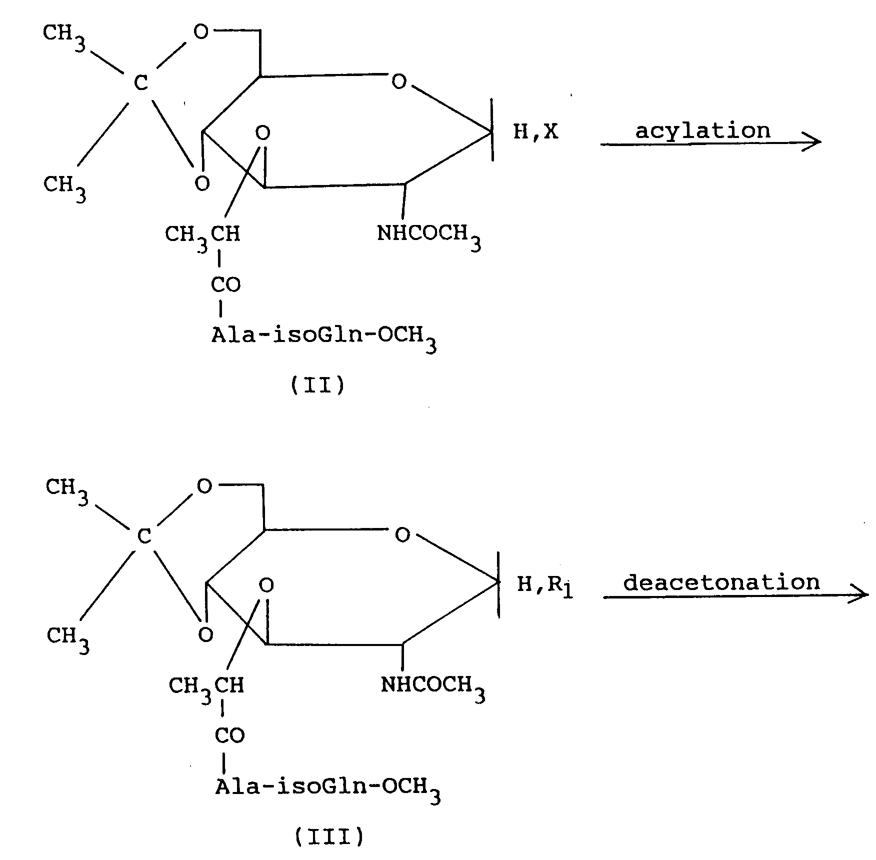 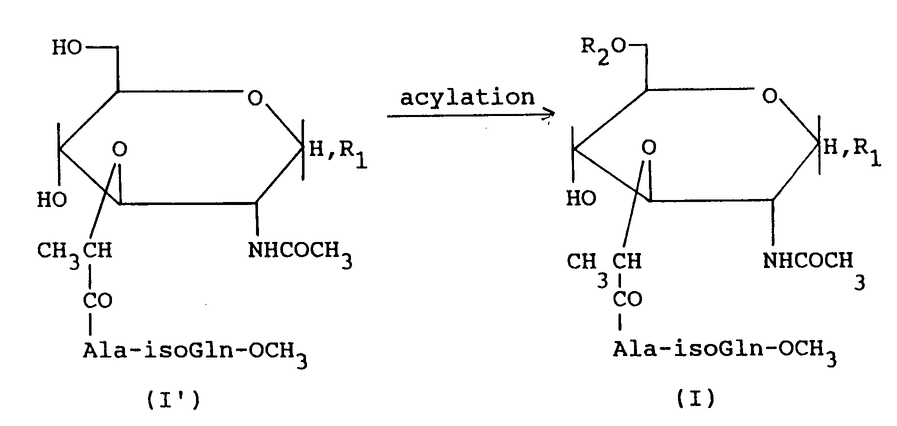
In the formulae, X is OH or SH; R₁ and R₂ are defined as above.

The above mentioned process consists of two acylation steps (the acylations at 6th and lst positions of the glucopyranose moiety) and one deacetonation step.

The two acylation steps can be conducted by reacting a compound of the formula (II) or (I′) with a specific acylating agent (R₂H, R₃H or its reactive derivative). These steps are generally carried out in an anhydrous organic solvent (for example, dimethylformamide or dioxane) and at room temperature or a slightly elevated temperature. When R₂H or R₃H (a free acid) is used, it is conducted in the presence of an appropriate condensing agent (for example, dicyclohexylcarbodiimide, N-cyclohexyl-N′-morpholinoethylcarbodiimide, N-cyclohexyl-N′-(4-diethylaminocyclohexyl)-carbodiimide or N,N′-diethylcarbodiimide). Examples of reactive derivatives of R₂H or R₃H are conventional reactive derivatives used in acylation, such as mixed acid anhydrides, active esters, acid halides and the like. The deacetonating step can be readily conducted under an acid hydrolysis condition (e.g., using 80% acetic acid aqueous solution) at a slightly elevated temperature.

The compounds of the formula (II) are known or can be readily prepared by known methods.

The compounds obtained by the above process may be purified by a conventional method such as a column chromatography using almina or silica gel, recrystallization and the like.

The compounds of the formula (I) of the present invention have an action for enhancing function of cells relevant to in vivo immune response and an action for increasing the number of said cells, and hence they are useful as an immunoregulating agent. The immunoregulating agent of the present invention can be used to enhance in vivo activities of vaccines such as BCG vaccine, hepatitis vaccine, influenza virus vaccine or the like, various antibacterial agents or anti-tumor agents.

The immunoregulating composition of the present invention comprises a compound of the formula (I) and a pharmaceutically acceptable carrier. The composition may be any dosage form for oral and parenteral administrations.

The compositions for oral administration are generally dosage forms such as powders, tablets, emulsions,capsules, granules and liquid prepartions (including liquid extracts, syrups and the like).

Examples of carriers for powders or other orally administrable solid preparations include lactose, starch, dextrin, calcium phosphate, calcium carbonate, synthetic or natural aluminium silicate, magnesium oxide, dried aluminium hydroxide, magnesium stearate, sodium bicarbonate, dried yeast and the like, and those for liquid preparations include water, glycerine, propylene glycol, simple syrup, ethanol, fatty oil, ethylene glycol, polyethylene glycol, sorbitol and the like. A typical example of the composition for parenteral administration is an injection. Liquid carriers for the injection include sterile distilled water. When a compound of the formula (I) is less soluble in water, an appropriate solubilizer is used. Each of the above preparations can be prepared by conventional methods.

When the compounds of the formula (II) of the present invention are used for enhancement of antitumor agents, they may be orally or parenterally administered to an adult human in an amount of 150 to 250»g/day in one dose. When used for enhancement of vaccines, they may be administered to an adult human in an amount of 0.5 to 2.0mg/1 to 2 weeks in one dose. For treatment of hepatitis, they may be orally or parenterally administered to an adult human 1 to 3 times for 3 months in an amount of 0.5 to 2.0mg in one dose. For enhancement of antibacterial agents, they may be used to an adult human in an amount 20 to 100»g/day in one dose.

The immunoregulating agents of the present invention may be generally used by formulating themselves only as described above. But they may be formulated together with an agent to be enhanced its action.

Further, the immunoregulating agents of the present invention can be used for not only humans but also other mammals such as pigs, bovines, sheeps, dogs, and cats.

The present invention is illustrated with following examples.

### Example 1

### N-[2-0-{2-Acetamido-2,3-dideoxy-1-0-(2-tetradecylhexadecanoyl)-α-D-glucopyranos-3-yl}-D-lactoyl]-L-alanyl-D-isoglutamine methylester

The compound of the formula (III) wherein R₁ is 2-tetradecylhexadecanoyloxy group (279.4mg, 0.281mmol) was dissolved in 80% acetic acid aqueous solution (8ml) and the resultant was allowed to stand for 2 hours at 45°C. After confirming the completion of the reaction with T.L.C.(CH₂Cl₂ : MeOH = 10 : 1), the resultant was concentrated under reduced pressure to obtain quantitatively the title compound (266.2mg).
mp : 147.0-148.0°C
[α]$\frac{\text{25}}{\text{D}}$: +44.38°(c=1.050, CH₂Cl : MeOH=1 : 1)
IR γmax(KBr)cm⁻¹ : 3350, 2930, 2850, 1740, 1650, 1520
NMR(CD₃OD-CHCl₃)δ(ppm) : 0.88(t,6H,J=6.6Hz), 1.26(s,48H), 1.38-1.43(m,6H), 1.51-1.62(m,4H), 1.93(s,3H), 3.70(s,3H), 6.16(d,1H,J=4.0Hz)

### Example 2

### N-{2-0-[2-Acetamido-2,3-dideoxy-1-S-(2-tetradecylhexadecanoyl)-1-thio-β-D-glucopyranos-3-yl]-D-lactoyl}-L-alanyl-D-isoglutamine methylester

The compound of the formula (III) wherein R₁ is 2-tetradecylhexadecanoylthio group (133.7mg) was dissolved in 80% acetic acid aqueous solution (15ml), which was allowed to react for 2 hours at 45°C. After confirming the completion of the reaction with T.L.C., the resultant was concentrated under reduced pressure and crystallized from ether to obtain quantitatively the title compound (127.0mg, crystals).
mp : 130.0-131.0°C
[α]$\frac{\text{25}}{\text{D}}$: +46.79°(c=1,201, CH₂Cl₂ : MeOH=1 : 1)
IRγ max(KBr)cm⁻¹ : 3300, 2920, 2850, 1720, 1630, 1530

### Example 3

### N-[2-0-{2-Acetamido-2,3-dideoxy-1-0-((3R)-3-tetradecanoyloxytetradecanoyl)-α-D-glucopyranos-3-yl}-D-lactoyl]-L-alanyl-D-isoglutamine methylester

The compound of the formula (III) wherein R₁ is 3-tetradecanoyloxytetradecanoyloxy group (409.1mg, 0.411mmol) was dissolved in 80% acetic acid aqueous solution (15ml) and allowed to stand for an hour at 45°C. In the same manner as that in Example 1, the title compound was quantitatively obtained (386.9mg).
mp : 133.8-134.6°C
[α]$\frac{\text{25}}{\text{D}}$: +44.74° (c=1.180, CHCl₃ : MeOH=1 : 1)
IR γmax(KBr)cm⁻¹ : 3700-3140, 2930, 2850, 1740, 1250, 1630, 1540
NMR(CDCl₃)δ : 0.89(t,6H,J=2.2Hz), 1.27(m,36H), 1.43(m,6H), 1.60(m,4H), 2.00(s,3H), 2.10-2.30(m,4H), 2.44-2.67(m,6H), 3.68(s,3H), 5.31(m,1H), 6.05(d,1H)

### Example 4

### N-[2-0-{2-Acetamido-2,3-dideoxy-1-S-((3R)-3-tetradecanoyloxytetradecanoyl)-1-thio-β-D-glucopyranos-3-yl}-D-lactoyl]-L-alanyl-D-isoglutamine methylester

The compound of the formula (III) wherein R₁ is 3-tetradecanoyloxytetradecanoylthio group (580.1mg, 0.5808mmol) was dissolved in 80% acetic acid aqueous solution (12ml) and allowed to stand for an hour at 45°C. After confirming the completion of the raction with T.L.C. (CH₂Cl₂: MeOH=10 : 1), the resultant was concentrated under reduced pressure. The resulting syrup was lyophilized to obtain quantitatively the title compound (555.2mg, crystals).
mp : 110-111°C
[α]$\frac{\text{25}}{\text{D}}$: +26.68°(c=0.787, CH₂Cl₂ : MeOH=2 : 1)
IR γmax(KBr)cm⁻¹ : 3650-3130, 3300, 2940, 2860, 1740, 1650, 1550,
NMR(CDCl₃-CD₃OD)δ : 0.88 (t,6H,J=6.6Hz), 1.25 (m,36H), 1.35 (d,3H,J=7.0Hz), 1.39 (d,3H,J=7.3Hz), 1.43-1.58 (m,4H), 1.93(s,3H), 1.93-2.04 (m,2H), 2.09-2.87(m,6H), 3.71(s,3H), 4.05(t,1H,J=10.4Hz), 4.28-4.33(m,1H), 4.31(q,1H,J=7.0Hz), 4.38-4.43 (m,1H), 5.12(d,1H,J=11.0Hz) 5.17-5.26(m,1H)

### Example 5

### N-[2-0-{2-Acetamido-2,3-dideoxy-6-0-decanoyl-1-0-(2-tetradecylhexadecanoyl)-α-D-glucopyranos-3-yl}-D-lactoyl]-L-alanyl-D-isoglutamine methylester

The compound of the formula (I) wherein R₁ is 2-tetradecylhexadecanoyloxy group (143.3mg, 0.150mmol) was dissolved in a mixture of dry dioxane (1.3ml) and dry N,N-dimethylformamide (DMF, 1.3ml). To the solution were added decanoic acid (29.6mg, 0.180mmol), dicyclohexylcarbodiimide (DCC, 61.7mg, 0.300mmol) and dimethylaminopyridine (DMAP, 9.1mg, 0.075mmol), and the resultant was stirred for 14 hours at room temperature. After the completion of the reaction, the reaction mixture was concentrated under reduced pressure. The resulting syrup was subjected to a column chromatography [Wakogel® C-200 eluted with CH₂Cl₂/MeOH ((a) 150 : 1 and (b) 20 : 1)] and the eluate eluted with the eluent (b) was concentrated under reduced pressure. The resultant syrup was subjected to a column chromatography [active alumina 90 eluted with CH₂Cl₂/MeOH ((a′) 150 : 1 and (b′) 20 : 1)], to remove DMAP and the eluate eluted with the eluent (b′) gave the title compound (121.3mg, yield: 72.6%).
mp : 116.3-117.0°C
[α]$\frac{\text{25}}{\text{D}}$: +42.56°(C=0.726, CHCl₃ : MeOH=2 : 1)
IR γmax(KBr)cm⁻¹ : 3650-3150, 2940, 2870, 1740, 1650, 1540
NMR(CDCl₃)δ : 0.88(t,6H,J=6.8Hz), 0.92(t,3H,J=7.1Hz), 1.25(m,62H), 1.39(d,3H,J=6.6Hz), 1.41(d,3H,J=6.6Hz), 1.51-1.60(m,6H), 1.90-2.23(m,2H), 1.94(s,3H), 2.33(t,2H,J-7.5Hz), 2.39-2.50(m,3H), 3.69(s,3H), 6.18(d,1H,J=3.7Hz)

### Example 6

### N-[2-0-{2-Acetamido-2,3-dideoxy-6-0-tetradecanoyl-1-0-(2-tetradecylhexadecanoyl)-α-D-glucopyranos-3-yl}-D-lactoyl]-L-alanyl-D-isoglutamine methylester

The compound of the formula (I′) wherein R₁ is 2-tetradecylhexadecanoyloxy group (139.7mg, 0.147mmol) is dissolved in a mixture of dry dioxane (2ml) and dry DMF (2ml). To the solution were added tetradecanoic acid (40.0mg, 0.176mmol), DCC(60.2mg, 0.294mmol) and DMAP(8.9mg, 0.074mmol). The resultant was stirred for 12 hours at room temperature and then treated in the same manner as that in Example 5 to obtain the title compound (105.4mg, yield: 61.7%).
mp : 116.8-117.7°C
[α]$\frac{\text{25}}{\text{D}}$: +29.22°(c=1.054, CH₂Cl₂)
IR γmax(KBr)cm⁻¹ : 3700-3100, 2940, 2860, 1740, 1660, 1540
NMR(CDCl₃)δ : 0.88(t,9H,J=6.4Hz), 1.25(m,7H), 1.38(d,3H, J=6.6Hz), 1.41(d,3H, J=7.3Hz), 1.49-1.60(m,6H), 1.93(s,3H), 2.03-2.21(m,2H), 2.32(t,2H,J=7.7Hz), 2.38-2.74(m,3H), 3.68(s,3H), 6.17(d,1H,J=3.7Hz)

### Esample 7

### N-[2-0-{2-Acetamido-2,3-dideoxy-6-0-octadecanoyl-1-0-(2-tetradecylhexadecanoyl)-α-D-glucopyranos-3-yl}-D-lactoyl]-L-alanyl-D-isoglutamine methylester

The compound of the formula (I′) wherein R₁ is 2-tetradecylhexadecanoyloxy group (122.7mg, 0.129mmol) was dissolved in a mixture of dry dioxane (1.5ml) and dry DMF(0.5ml). To the solution were added octadecanoic acid (44.5mg, 0.155mmol), DCC(53.7mg, 0.258mmol) and DMAP(7.9mg, 0.065mmol). The resultant was stirred for 14 hours and then treated in the same manner as that in Example 5 to obtain the title compound(119.0mg, yield: 75.6%).
mp : 118.7-120.0°C
[α]$\frac{\text{25}}{\text{D}}$: +39.45°(c=0.621, CHCl₃ : MeOH=2 : 1)
IR γmax(KBr)cm⁻¹ : 3650-3150, 2930, 2860, 1740, 1650, 1540
NMR(CDCl₃)δ : 0.88(t,9H,J=6.6Hz), 1.25(m,78H), 1.39(d,3H,J=6.6Hz), 1.41(d,3H,J=6.6Hz), 1.49-1.60(m,6H), 1.94(s,3H), 1.90-2.26(m,2H), 2.32(t,2H,J=7.3Hz), 2.39-2.50(m,3H), 3.69(s,3H), 6.18(d,1H,J=3.7Hz)

### Example 8

### N-[2-0-{2-Acetamido-2,3-dideoxy-6-0-decanoyl-1-S-(2-tetradecylhexadecanoyl)-1-thio-β-D-glucopyranos-3-yl}-D-lactoyl]-L-alanyl-D-isoglutamine methylester

The compound of the formula (I′) wherein R₁ is 2-tetradecylhexadecanoylthio (128.4mg, 0.134mmol) was dissolved in a mixture of dry dioxane (1.5ml) and dry DMF (1.0ml). To the solution were added decanoic acid (27.4mg, 0.161mmol), DCC(54.6mg, 0.268mmol) and DMAP(8.1mg, 0.067mmol). The resultant was stirred for 6.5 hours at room temperature. After confirming the completion of the reaciton with T.L.C. (10 : 1), the resultant was lyophilized and subjected to a column chromatography [Wakogel® C-200 eluted with CH₂Cl₂/MeOH ((a) 150 : 1 and (b) 50 : 1)]. The eluate eluted with the eluent (b) gave the title compound (99.6mg, yield: 66.8%).
mp : 98.6-99.4°C
[α]$\frac{\text{25}}{\text{D}}$: +17.69°(c=0.797, CH₂Cl₂: MeOH=2 : 1)
IR γmax(KBr)cm⁻¹ : 3500-3200, 2950, 2880, 1750, 1640, 1560
NMR(CDCl₃-CD₃OD)δ : 0.88(t,6H,J=6.6Hz), 0.92(t,3H,J=7.1Hz), 1.25(m,56H), 1.34(d,3H,J=6.6Hz), 1.40(d,3H,J=7.0Hz), 1.58-1.71(m,6H), 1.90(s,3H), 1.94-2.24(m,2H), 2.33(t,2H,J=7.5Hz), 2.42-2.52(m,3H), 3.69(s,3H), 5.11(d,1H,J=10.6Hz)

### Example 9

### N-[2-0-{2-Acetamido-2,3-dideoxy-6-0-tetradecanoyl-1-S-(2-tetradcylhexadecanoyl)-1-thio-β-D-glucopyranos-3-yl}-D-lactoyl]-L-alanyl-D-isoglutamine methylester

The compound of the formula (I′) wherein R₁ is 2-tetradecylhexadecanoylthio group (125.0mg, 0.131mmol) was dissolved in a mixture of dry dioxane (1.5ml) and dry DMF (1.0ml). To the solution were added tetradecanoic acid (35.3mg, 0.157mmol), DCC(53.1mg, 0.262mmol) and DMAP (7.9mg, 0.066mmol). The resultant was stirred for 3 hours at room temperature and then treated in the same manner as that in Example 8 to obtain the title compound (125.8mg, yield: 82.5%).
mp : 99.0-100.4°C
[α]$\frac{\text{25}}{\text{D}}$: +2.13°(c=2.16, CH₂Cl₂)
IR γmax(KBr)cm⁻¹ : 3650-3200, 2930, 2860, 1740, 1650, 1550
NMR(CDCl₃)δ : 0.85-0.95(m,9H), 1.25(m,68H), 1.36(d,3H,J=6.6Hz), 1.41(d,3H,J=7.3Hz), 1.47-1.76(m,6H), 1.88-2.32(m,2H), 1.94(s,3H), 2.35(t,2H,J=7.9Hz), 2.47-2.53(m,3H), 3.70(s,3H), 5.12(d,1H,J=10.3Hz)

### Example 10

### N-[2-0-{2-Acetamido-2,3-dideoxy-6-0-octadecanoyl-1-S-(2-tetradecylhexadecanoyl)-1-thio-β-D-glucopyranos-3-yl}-D-lactoyl]-L-alanyl-D-isoglutamine methylester

The compound of the formula (I′) wherein R₁ is 2-tetradecylhexadecanoylthio (122.4mg, 0.128mmol) was dissolved in a mixture of dry dioxane (1.5ml) and dry DMF (1.0ml). To the solution were added octadecanoic acid (43.0mg, 0.145mmol), DCC(52.0mg, 0.245mmol) and DMAP (7.7mg, 0.064mmol). The resultant was stirred for 4 hours at room temperature and treated in the same manner as that in Example 8 to obtain the title compound (102.7mg, yield : 65.6%).
mp : 99.3-101.0°C
[α]$\frac{\text{25}}{\text{D}}$: +2.06° (c=0.376, CH₂Cl₂ MeOH=2 : 1)
IR γmax(KBr)cm⁻¹ : 3600-3150, 2920, 2840, 1740, 1640, 1540
NMR(CDCl₃-CD₃OD)δ : 0.88(t,9H,J=6.6Hz), 1.26(m,76H), 1.35(d,3H,J=6.6Hz), 1.40(d,3H,J=7.3Hz), 1.58-1.61(m,6H), 1.88(s,3H), 1.92-2.26(m,2H), 2.33(t,2H,J=7.7Hz), 2.41-2.55(m,3H), 3.69(s,3H), 4.40(q,1H), 5.10(d,1H,J=11.0Hz)

### Example 11

### N-[2-0-{2-Acetamido-2,3-dideoxy-6-0-decanoyl-1-0-((3R)-3-tetradecanoyloxytetradecanoyl)-α-D-glucopyranos-3-yl}-D-lactoyl]-L-alanyl-D-isoglutamine methylester

The compound of the formula (I′) wherein R₁ is 3-tetradecanoyloxytetradecanoyloxy group (116.9mg, 0.121mmol) was dissolved in a mixture of dioxane (1.5ml) and dry DMF (0.5ml). To the solution were added decanoic acid (24.3mg, 0.145mmol), DCC (48.4mg, 0.242mmol) and DMAP (7.2mg, 0.161mmol). The resultant was stirred for 8 hours at room temeperature. After confirming, the completion of the reaction with T.L.C. (CH₂Cl₂ MeOH=10 : 1), the resultant was concentrated under reduced pressure. The resulting syrup was subjected to a column chromatography [Wakogel® C-200 eluted with CH₂Cl₂/MeOH ((a) 150 : 1 and (b) 35 : 1)]. The eluate eluted with the eluent (b) gave the title compound (80.4mg, yield : 59.6%).
mp : 72.0-72.8°C
[α]$\frac{\text{25}}{\text{D}}$ : +27.86°(c=0.804, CH₂Cl₂)
IR γmax(Film)cm⁻¹ : 3700-3100, 2930, 2850, 1740, 1650, 1540
NMR(CDCl₃)δ : 0.88(t,9H,J=6.6Hz), 1.25-1.27(m,48H), 1.43(d,3H, J=6.6Hz), 1.45(d,3H,J=7.0Hz), 1.61(m,6H), 2.00(s,3H), 2.04-2.24(m,2H), 2.30(t,2H, J=7.5Hz), 2.32-2.67(m,6H), 3.69(s,3H), 4.21(q,1H,J=6.6Hz), 5.29(m,1H), 6.05(d,1H,J=3.3Hz)

### Example 12

### N-[2-0-{2-Acetamido-2,3-dideoxy-6-0-tetradecanoyl-1-0-((3R)-3-tetradecanoyloxytetradecanoyl)-α-D-glucopyranos-3-yl}-D-lactoyl]-L-alanyl-D-isoglutamine methylester.

The compound of the formula (I′) wherein R₁ is 3-tetradecanoyloxytetradecanoyloxy (108.6mg, 0.115mmol) was dissolved in a mixture of dry dioxane (1.5ml) and dry DMF (0.5ml). To the solution were added tetradecanoic acid (30.1mg, 0.137mmol), DCC(45.3mg, 0.228mmol) and DMAP (6.7mg, 0.057mmol). The mixture was allowed to react for 14 hours at room temperature. The resultant was treated in the same manner as that in Example 11 to obtain the title compound (99.1mg, yield: 74.6%).
mp : 72.5-73.6°C
[α]$\frac{\text{25}}{\text{D}}$: +26.51°(c=1.388,CH₂Cl₂)
IR γmax(film)cm⁻¹ : 3700-3150, 2930, 2850, 1740, 1660, 1540
NMR(CDCl₃)δ : 0.88(t,9H,J=6.6Hz), 1.25-1.38(m,56H), 1.43(d,3H,J=6.6Hz), 1.45(d,3H,J=7.0Hz), 1.60(m,6H), 1.99(s,3H), 2.06-2.24(m,2H), 2.30(t,2H,J=7.5Hz), 2.32-2.66(m,6H), 3.69(s,3H), 4.21(q,1H,J=7.0Hz), 5.30(m,1H), 6.05(d,1H,J=3.3Hz)

### Example 13

### N-[2-0-{2-Acetamido-2,3-dideoxy-6-0-octadecanoyl-1-0-((3R)-3-tetradecanoyloxytetradecanoyl)-α-D-glucopyranos-3-yl}-D-lactoyl]-L-alanyl-D-isoglutamine methylester

The compound of the formula (I′) wherein R₁ is 3-tetradecanoyloxytetradecanoyl group (108.8mg, 0.114mmol) was dissolved in a mixture of dry dioxane (1.5ml) and dry DMF (0.5ml). To the solution were added octadecanoic acid (37.6mg, 0.137mmol), DCC (45.4mg, 0.228mmol) and DMAP (6.7mg, 0.057mmol). The resultant was stirred for 14 hours at room temperature and then treated in the same manner as that in Example 11 to obtain the title compound (95.6mg, yield: 68.5%).
mp : 68.1-69.0°C
[α]$\frac{\text{25}}{\text{D}}$: +26.15°(c=1.338, CH₂Cl₂)
IR γmax(film)cm⁻¹ : 3700-3150, 2930, 2850, 1740, 1650, 1540
NMR(CDCl₃)δ : 0.88(t,9H,J=6.6Hz), 1.25-1.39(m,64H), 1.43(d,3H,J=6.6Hz), 1.44(d,3H,J=7.0Hz), 1.58-1.60(m,6H), 1.99(s,3H), 2.02-2.22(m,2H), 2.30(t,2H,J=7.5Hz), 2.32-2.67(m,6H), 3.69(s,3H), 4.21(q,1H,J=6.6Hz), 5.30(m,1H), 6.05(d,1H,J=3.3Hz)

### Example 14

### N-[2-0-{2-Acetamido-2,3-dideoxy-6-0-decanoyl-1-S-((3R)-R-tetradecanoyloxytetradecanoyl)-1-thio-β-D-glucopyranos-3-yl}-D-lactoyl]-L-alanyl-D-isoglutamine methylester

The compound of the formula (I) wherein R₁ is 3-tetradecanoyloxytetradecanoylthio (239.2mg, 0.250mmol) was dissolved in a mixture of dry dioxane (0.5ml) and dry DMF (0.5ml). To the solution were added decanoic acid (51.6ml, 0.300mmol), DCC (102.9mg, 0.50mmol) and DMAP (15.2mg, 0.499mmol), and the resultant was stirred for 2 hours at room temperature. After confirming the completion of the reaction with T.L.C. (CH₂Cl₂ MeOH=10 : 1), DC urea of a reaction by-product was filtered off and washed with dioxane. The filtrate and washings were combined and then lyophilized. The amorphous material thus obtained was subjected to a column chromatography [Wakogel® C-200 eluted with CH₂Cl₂/MeOH ((a) 200 : 1, (b) 70 : 1, (c) 60 : 1 and (d) 40 : 1)]. The eluate eluted with the eluent (c) gave the title compound (111.6mg, yield: 40.2%).
mp : 138.6 - 139.9°C
[α]$\frac{\text{25}}{\text{D}}$ : +17.09° (c=0.702, CH₂Cl₂ : MeOH = 2:1)
IRγmax(film)cm⁻¹ : 3650-3020, 3250, 2930, 2850, 1740, 1660, 1540
NMR(CDCl₃)δ: 0.87(t,9H,J=5.7Hz), 1.25(m,52H), 1.39(d,3H,J=6.6Hz), 1.58(m,6H), 1.95(s,3H), 2.10-2.28(m,2H), 2.34(6,2H,J=7.7Hz), 2.47-2.91(m,6H), 3.69(s,3H), 5.13(d,1H,J=11.0Hz), 5.11-5.21(m,1H)

### Example 15

### N-[2-0-{2-Acetamido-2,3-dideoxy-6-0-tetradecanoyl-1-S-((3R)-3-tetradecanoyloxytetradecanoyl)-1-thio-β-D-glucopyranos-3-yl}-D-lactoyl]-L-alanyl-D-isoglutamine methylester

The compound of the formula (I′) wherein R₁ is 3-tetradecanoyloxytetradecanoylthio (206.5mg, 0.215 mmol) was dissolved in dry dioxane (0.5ml) and dry DMF (0.5ml). To the solution were added tetradecanoic acid (59.0mg, 0.259 mmol), DCC (88.9mg, 0.431 mmol) and DMAP (13.1mg, 9.1077 mmol). The resultant was stirred for 2.5 hours at room temperature and then treated in the same manner as that in Example 14 to obtain the title compound (95.6mg, yield: 38.0%).
mp : 136.1 - 137.7°C
[α]$\frac{\text{25}}{\text{D}}$ : +17.57°(c=0.956, CH₂Cl₂:MeOH = 2:1)
IR γmax (film)cm⁻¹: 3650-3120, 3300, 2930, 2860, 1740, 1640, 1540
NMR(CDCl₃)δ: 0.88(t,9H,J=6.6Hz), 1.25(m,56H), 1.39(d,3H,J=7.0Hz), 1.42(d,3H,J=7.0Hz), 1.57(m,6H), 1.97(s,3H), 2.01-2.28(m,2H), 2.34(t,2H,J=7.7Hz), 3.71(s,3H), 5.13(d,1H,J=11.0Hz), 5.11-5.23(m,1H)

### Example 16

### N-[2-0-{2-Acetamido-2,3-dideoxy-6-0-octadecanoyl-1-S-((3R)-3-tetradecanoyloxytetradecanoyl)-1-thio-β-D-glucopyranos-3-yl}-D-lactoyl]-L-alanyl-D-isoglutamine methylester

The compound of the formula (I′) wherein R₁ is 3-tetradecanoyloxytetradecanoylthio (203.2mg, 0.212 mmol) was dissolved in a mixture of dry dioxane (0.5ml) and dry DMF (0.5ml). To the solution were added octadecanoic acid (63.1mg, 0.254 mmol), DCC (87.5mg, 0.424 mmol) and DMAP (12.9mg, 0.106 mmol). The resultant was stirred for 3 hours at room temperature and then treated in the same manner as that in Example 14 to obtain the title compound (112.2mg, yield: 43.2%).
mp : 133.7 - 134.5°C
[α]$\frac{\text{25}}{\text{D}}$: +17.46° (c=1.122, CH₂Cl₂:MeOH = 2:1)
IR γmax(film)cm⁻¹ : 3700-3150, 3320, 2960, 2900, 1750, 1680, 1580
NMR(CDCl₃)δ: 0.87(6,9H,J=5.5Hz), 1.25(m,6.6H), 1.39(d,3H,J=5.9Hz), 1.57(m,6H), 1.95(s,3H), 1.95-2.18(m,2H), 2.25-2.90(m,6H), 2.38(t,2H,J=7.1Hz), 3.69(s,3H), 5.13(d,1H,J=11.0Hz), 5.11-5.20(m,1H)

### Example 17

### N-[2-0-{2-Acetamido-2,3-dideoxy-1-0-((3R)-3-hexadecanoyloxytetradecanoyl)-α-D-glucopyranos-3-yl}-D-lactoyl]-L-alanyl-D-isoglutamine methylester

The compound of the formula (III) wherein R₁ is 3-hexadecanoyloxytetradecanoyloxy (408.1mg, 0.410 mmol) was dissolved in 80% acetic acid aqueous solution (15ml), which was allowed to stand for 1.5 hours at 45°C. In the same manner as that in Example 4, the title compound (391.7mg) was quantitatively obtained from the above solution.
m.p. : 134.2 - 135.5°C
[α]$\frac{\text{25}}{\text{D}}$: +47.38° (c=0.878, CH₂Cl₂:MeOH = 1:1)
IR γmax (cm)⁻¹ : 3700-3100(OH), 3300(NH) 2920, 2850(CH) 1740(ester) 1650, 1530(amido)
NMR(CDCl₃)δ : 0.88(t,9H,JMeCH₂6Hz,3MeCH₂), 1.25(m,40H,20CHz), 1.42(d,3H,J_{MeCH}7.3Hz,MeC of Ala), 1.45(d,3H,J_{MeCH}7.3Hz,HeC of Lac), 1.57-1.60(m,6H,3MeCh₂), 1.95-2.17(m,2H,CH₂CH of Gln), 2.00(S,3H,AcN), 2.30(t,2H,JCH₂CH₂7.5Hz,CH₂CO of Gln), 2.37-2.66(m,6H,3CH₂CO), 3.68(S,3H,COOMe), 5.30-5.42(m,1H,H-3 of C₁₇-O-C₁₆), 6.03(d,1H,J_{1.2},3.3Hz,H-1),

### Example 18

### N-[2-0-{2-Acetamido-2,3-dideoxy-6-0-octadecanoyl-1-0-((3R)-3-hexadecanoyloxytetradecanoyl)-α-D-glucopyranos-3-yl}-D-lactoyl]-L-alanyl-D-isoglutamine methylester

The compound of the formula (I′) wherein R₁ is 3-hexadecanoyloxytetradecanoyloxy (191.1mg, 0.200mmol) was dissolved in a mixture of dry dioxane (3.0ml) and dry DMF (1.0ml). To the solution were added octadecanoic acid (74.0mg, 0.260mmol), DDC(82.5mg, 0.400mmol) and DMAP (12.2mg, 0.100mmol). The resultant was stirred for 16 hours at room temperature. In the same manner as that in Example 11, the title compound (193.1mg, yield : 78.9%) was obtained.
mp : 69.5-71.0°C
[α]$\frac{\text{25}}{\text{D}}$: +40.69°(C=1.504, CH₂Cl₂ : MeOH=2 : 1)
IR γmax(cm⁻¹): 3700-3130(OH), 3300(NH), 2930, 2860(CH), 1740(ester), 1660, 1540(amido),
NMR(CDCl₃) : 0.88(t,9H,JMeCH₂6.6Hz,3MeCH₂), 1.25(m,68H,34CH₂), 1.43(d,3H,JMeCH5.9Hz,MeC of Ala), 1.45(d,3H,JMeCH5.9Hz,MeC of Lac), 1.60(m,6H,3MeCH₂), 1.99(s,3H,AcN), 2.19-2.66(m,8H,CH₂CH of Gln,3CH₂CO), 2.35(t,2H,JCH₂CH₂7.7Hz,CH₂CO of Gln), 3.69(s,3H,COOMe), 5.30(m,1H,H-3 of C₁₄OC₁₆), 6.05(d,1H,J_{1.2}, 2.9Hz,H-1)

### Example 19

### N-[2-0-{2-Acetamido-2,3-dideoxy-1-S-((3R)-3-hexadecaonyloxytetradecanoyl)-1-thio-β-D-glucopyranos-3-yl}-D-lactoyl]-L-alanyl-D-isoglutamine methylester

The compound of the formula (III) wherein R₁ is 3-hexadecanoyloxytetradecanoylthio (586.6mg, 0.580mmol) was dissolved in 80% acetic acid aqueous solution (12ml), which was allowed to stand for an hour at 45°C. In the same manner as that in Example 4, the title compound (563.4mg) was quantitatively obtained.
mp : 94.6-95.8°C
[α]$\frac{\text{25}}{\text{D}}$: +28 06(C=1.112, CH₂Cl₂. : MeOH=1 : 1)
IR γmax(cm⁻¹) : 3680-3130(OH), 3300(NH), 2940, 2870(CH), 1740(ester), 1650, 1550(amido)
NMR(CDCl₃) : 0.88(t,6H,JMeCH₂6.4Hz,2MeCH₂), 1.27(m,40H,20CH₂), 1.37(d,3H,JMeCH7.0Hz,MeC of Lac), 1.41(d,3H,JMeCH7.0Hz,MeC of Ala), 1.60(m,4H,2MeCH₂), 1.91(s,3H,AcN), 1.91-2.02(m,1H,CHCH₂ of Gln), 2.21-2.90(m,6H,CH₂CO of Gln,2CH₂CO), 3.70(s,3H,COOMe), 4.05(t,1H,J_{6a,6b},10.3Hz,H-6a), 4.22-4.26(m,2H,CH of Lac and Ala), 4.34-4.39(m,1H,CH of Gln), 5.13(d,1H,J_{1.2}10.6Hz,H-1), 5.21-5.25(m,1H,H-3 of C₁₄OC₁₆)

### Example 20

### N-[2-0-{2-Acetamldo-2,3-dideoxy-6-0-octadecanoyl-1-S-((3R)-3-hexadecanoyloxytetradecanoyl)-1-thio-β-D-glucopyranos-3-yl}-D-lactoyl]-L-alanyl-D-isoglutamine methylester

The compound of the formula (I′) wherein R₁ is 3-hexadecanoyloxytetradecanoylthio(340.0mg, 0.350mmol) was dissolved in a mixture of dry dioxane (4.0ml) and dry DMF(1.5ml). To the solution were added octadecanoic acid(129.4mg, 0.455mmol), DCC(144.4mg, 0.700mmol) and DMAP(21.4mg, 0.175mmol). The resultant was stirred for 3 hours at room temperature. In the same manner as that in Example 14, the title compound (203.1mg, yield : 46.8%) was obtained.
mp : 171.2-172.8°C
[α]$\frac{\text{25}}{\text{D}}$: +17.01°(C=0.723, CH₂Cl₂ : MeOH=2 : 1)
IR γmax(cm⁻¹) : 3320, 3270(NH, OH), 2920, 2850(CH), 1740(ester), 1650, 1540(amido)
NMR(CDCl₃) : 0.87(t,9H,JMeCH5.3Hz,3MeCH₂), 1.25(m,70H,35CH₂), 1.39(d,3H,JMeCH6.6Hz,MeC of Ala), 1.58(m,6H,3MeCH₂), 1.94(s,3H,AcN), 2.22-2.91(m,2H,CH₂CO of Gln), 2.32(t,2H,JCH₂CH₂7.7Hz,CH₂CO of Gln), 3.69(s,3H,COOMe), 5.13(d,1H,J_{1.2}11.0Hz,H-1), 5.11-5.20(m,1H,H-3 of C₁₄-O-C₁₆)

### Example 21

### N-[2-0-{2-Acetamido-2,3-dideoxy-1-0-((3R)-3-octadecanoyloxytetradecanoyl)-α-D-glucopyranos-3-yl}-D-lactoyl]-L-alanyl-D-isoglutamine methylester

The compound of the formula (III) wherein R₁ is 3-octadecanoyloxytetradecanoyloxy(388.9mg, 0.380mmol) was dissolved in 80% acetic acid aqueous solution(15ml), which was allowed to stand for 2 hours at 45°C. In the same manner as that in Example 4, the title compound (373.7mg) was quantitatively obtained(373.7mg).
mp : 187-188.5°C
[α]$\frac{\text{25}}{\text{D}}$: +47.11°(C=0.900, CH₂Cl₂ : MeOH=1 : 1)
IR γmax(cm⁻¹) : 3700-3100(OH), 3300(NH), 2910, 2850(CH), 1740(ester), 1650, 1540(amido)
NMR(CDCl₃) : 0.88(t,9H,JMe₁CH₂7.0Hz,3MeCH₂), 1.25(m,44H,22CH₂), 1.41(d,3H,JMeCH7.8Hz,MeC of Ala), 1.44(d,3H,JMeCH7.8Hz,MeC of Lac), 1.99(s,3H,AcN), 1.94-2.04(m,2H,CH₂CH of Gln), 2.30(t,2H,JCH₂CH₂8.0_{z},CH₂CO of Gln), 2.27-2.46(m,6H,3CH₂CO), 3.70(s,3H,COOMe), 5.30(m,1H,H-3 of C₁₄OC₁₈), 6.05(d,1H,J₁₂7.8Hz,H-1)

### Example 22

### N-[2-0-{2-Acetamido-2,3-dideoxy-6-0-octadecanoyl-1-0-((3R)-3-octadecanoyloxytetradecanoyl)-α-D-glucopyranos-3-yl}-D-lactoyl]-L-alanyl-D-isoglutamine methylester

The compound of the formula (I′) wherein R₁ is 3-octadecanoyloxytetradecanoyloxy(177,0mg, 0.18mmol) was in a mixture of dry dioxane(3.0ml) and dry DMF(1.0ml). To the solution were added octadecanoic acid(66.6mg, 0.234mmol), DCC(74.3mg, 0.360mmol) and DMAP(11.0mg, 0.090mmol). The resultant was stirred for 16 hours at room temperature. In the same manner as that in Example 11, the title compound (164.0mg, 72.8%) was obtained.
mp : 106-108.5°C
[α]$\frac{\text{25}}{\text{D}}$: +38.46°(C=0.624, CH₂Cl₂ : MeOH=1 : 1)
IR γmax(cm⁻¹) : 3700-3150(H), 3300(NH), 2930, 2860(CH), 1740(ester), 1660, 1540(amido)
NMR(CDCl₃) : 0.88(t,9H,JCH₂CH₂66Hz,3MeCH₂), 1.25(m,72H,36CH₂), 1.43(d,3H,JMeCH6.2Hz,MeC of Lac), 1.59(m,6H,3MeCH₂), 1.99(s,3H,AcN), 2.01-2.20(m,8H,CH₂CH of Gln,3CH₂CO), 2.30(t,2H,JCH₂CH₂7.7Hz,CH₂CO of Gln), 3.69(s,3H,COOMe), 5.30(m,1H,H-3 of C₁₄OC₁₈), 6.05(d,1H,J_{1.2}3.0Hz,H-1)

### Example 23

### N-[2-0-{2-Acetamido-2,3-dideoxy-1-S-((3R)-3-octadecanoyloxytetradecanoyl)-1-thio-β-D-glucopyranos-3-yl}-D-lactoyl]-L-alanyl-D-isoglutamine methylester

The compound of the formula (III) wherein R₁ is 3-octadecanoyloxytetradecanoylthio(634.1mg, 0.610mmol) was dissolved in 80% acetic acid acqueous solution(15ml), which was allowed to stand for an hour at 45°C. In the same manner as that in Example 4, the title compound (609.6mg) was quantitatively obtained.
mp : 112.5-113.8°C
[α]$\frac{\text{25}}{\text{D}}$: +24.62°(C=0.600, Ch₂Cl₂ : MeOH=1 : 1)
IR γmax(cm⁻¹) : 3400-3100(OH), 3260(NH), 2910, 2850(CH), 1740(ester), 1640, 1530(amido)
NMR(CDCl₃) : 0.88(t,6H,JMeCH₂,4.0Hz,2MeCH₂), 1.25(m,44H,22CH₂), 1.33(d,3H,JMeCH7.3Hz,MeC of Lac), 1.36(d,3H,JMeCH7.3Hz,MeC of Ala), 1.58(m,4H,2MeCH₂), 1.92(s,3H,AcN), 1.91-2.04(m,2H,CHCH₂ of Gln), 2.26(m,6H,CH₂CO of Gln,2CH₂CO), 3.69(s,3H,COOMe), 4.02-4.09(t,1H,J_{6a,6b}8.7Hz,H-6a), 4.28(m,1H,CH of Gln), 5.08(d,1H,J_{1.2}4.0Hz,H-1), 5.21(m,1H,H-3 of C₁₄-O-C₁₈)

### Example 24

### N-[2-0-{2-Acetamido-2,3-dideoxy-6-0-octadecanoyl-1-S-((3R)-3-octadecanoyloxytetradecanoyl)-1-thio-β-D- glucopyranos-3-yl}-D-lactoyl]-L-alanyl-D-isoglutamine methylester

The compound of the formula (I′) wherein R₁ is 3-octadecanoyloxytetradecanoylthio(400.0mg, 0.400mmol) was dissolved in a mixture of dry dioxane (4.0ml) and dry DMF(1.5ml). To the solution were added octadecanoic acid(147.9mg, 0.520mmol), DCC(165.1mg, 0.800mmol) and DMAP(24.4mg, 0.200mmol), and the resultant was stirred for 3.5 hours at room temperature. In the same manner as that in Example 14, the title compound (258.6mg, yield : 51.0%) was obtained.
mp : 123.1-124.5°C
[α]$\frac{\text{25}}{\text{D}}$: +17.01°(C=0.723, CH₂Cl₂ : MeOH=2 : 1)
IR γmax(cm⁻¹) : 3650-3150(OH), 3300(NH), 2930, 2850(CH), 1730(ester), 1650, 1550(amido)
NMR(CDCl₃) : 0.88(t,9H,JMeCH₂5.5Hz,3MeCH₂), 1.25(m,74H,37CH₂), 1.34(d,3H,JMeCH6.6Hz,MeC of Ala), 1.44-1.67(m,6H,3MeCH₂), 1.85(s,3H,AcN), 2.17-2.79(m,2H,CH₂CHof Gln), 2.31(t,2H,JCHh₂CH₂8.4Hz,CH₂CO of Gln), 3.70(s,3H,COOMe), 5.10(d,1H,J_{1.2}10.6Hz,H-1), 5.14(m,1H,H-3 of C₁₄OC₁₈)

### Example 25

### N-[2-0-{2-Acetamido-2,3-dideoxy-1-0-((3R)-3-tetracosanoyloxytetradecanoyl)-α-D-glucopyranos-3-yl}-D-lactoyl]-L-alanyl-D-isoglutamine methylester

The compound of the formula (III) wherein R₁ is 3-tetracosanoyloxytetradecanoyloxy(465.2mg, 0.420mmol) was dissolved in 80% acetic acid aqueous solution (15ml), which was allowed to stand for an hour at 45°C. In the same manner as that in Example 4, the title compound(448.3mg) was quantitatively obtained.
mp : 183.5-185°C
[α]$\frac{\text{25}}{\text{D}}$: +34.13° (C=0.920, CH₂Cl₂ : MeOH=2 : 1)
IR γmax(cm⁻¹) : 3700-3120(OH), 3300(NH), 2930, 2850(CH), 1740(ester), 1660, 1540(amido)
NMR(CDCl₃) : 0.89(t,9H,JMeCH₂6.6Hz,3MeCH₂), 1.25(m,56H,28CH₂), 1.41(d,3H,JMeCH6.9Hz,MeC of Ala), 1.44(d,3H,JMeCH6.9Hz,MeC of Lac), 1.60(m,6H,3MeCH₂), 1.99(s,3H,AcN), 1.94-2.01(m,2H,CH₂CHof Gln), 2.20-2.39(m,6H,3CH₂CO), 2.29(t,2H,JCH₂CH₂13Hz,CH₂OO of Gln), 3.69(s,3H,COOMe), 5.30(m,1H,H-3 of C₁₄OC₂₄), 6.05(d,1H,J_{1.2}2.9Hz,H-1)

### Example 26

### N-[2-0-{2-Acetamido-2,3-dideoxy-6-0-octadecanoyl-1-0-((3R)-3-tetracosanoyloxytetradecanoyl)-α-D-glucopyranos-3-yl}-D-lactoyl]-L-alanyl-D-isoglutamine methylester

The compound of the formula (I′) wherein R₁ is 3-tetracosanoyloxytetradecanoyloxy (234.8mg, 0.220mmol) was dissolved in a mixture of dry dioxane (3.0ml) and dry DMF(1.0ml). To the solution were added octadecanoic acid (81.3mg, 0.286mmol), DCC (90.8mg, 0.440mmol) and DMAP(13.4mg, 0.110mmol), and the resultant was stirred for 12 hours at room temperature. In the same manner as that in Example 11, the title compound (200.7mg, yield : 68.3%) was obtained.
mp : 66.5-68°C
[α]$\frac{\text{25}}{\text{D}}$: +34.11° (c=0.680, CH₂Cl₂ : MeOH=1 : 1)
IR γmax(cm⁻¹) : 3700-3100(OH), 3300(NH), 2930, 2850(CH), 1760(ester), 1660, 1540(amido)
NMR(CDCl₃) : 0.88(t,9H,JCH₂CH₂6.6Hz,3MeCH₂), 1.25(m,88H,44CH₂), 1.43(d,3H,JMeOH6.6Hz,MeC of Ala), 1.44(d,3H,JMeOH7.0Hz,MeC of Lac), 1.61(m,6H,3MeCH₂), 2.00(m,8H,CH₂CH of Gln,3CH₂CO), 1.99(s,3H,AcN), 2.33(t,2H,JCH₂CH₂4.2Hz,CH₂CO of Gln). 3.65(s,3H,COOMe), 4.20(q,1H,JMe₁CH,Hz,MeCH of Ala), 5.29(m,1H,H-3 of C₁₄OC₂₄), 6.04(d,1H,J_{1.2}33Hz,1-H)

### Example 27

### N-[2-0-{2-Acetamido-2,3-dideoxy-1-S-((3R)-3-tetracosanoyloxytetradecanoyl)-1-thio-β-D-glucopyranos-3-yl}-D-lactoyl]-L-alanyl-D-isoglutamine methylester

The compound of the formula (III) wherein R₁ is 3-tetracosanoyloxytetradecanoylthio (617.9mg, 0.550mmol) was dissolved in 80% acetic acid aqueous solution (12ml), which was allowed to stand for an hour at 45°C. In the same manner as that in Example 4, the title compound (595.9mg) was quantitatively obtained.
mp : 168.5-170.1°C
[α]$\frac{\text{25}}{\text{D}}$: +5.18° ( C=0.772, CH₂Cl₂ MeOH=1 : 1)
IR γmax(cm⁻¹) : 3500-3200(OH), 3280(NH), 2910, 2850(CH), 1720(ester), 1630, 1540(amido)
NMR(CDCl₃) : 0.88(t,6H,JMe₁CH₂ Hz,2MeCH₂) 1.25(m,56H,28CH₂), 1.30(d,3H,JMeCH8.3Hz,MeC of Lac), 1.34(d,3H,JMeCH7.5Hz,MeC of Ala), 1.58(m,4H,2MeCH₂), 1.97-1.91(m,2H,2MeCH₂), 1.94(s,3H,AcN), 2.25(m,6H,CHCH₂ of Gln), 3.69(s,3H,COOMe), 4.05(t,1H,J_{6a,6b}10.1Hz,H-6a), 4.24-4.28(m,1H,CH of Gln), 5.09(d,1H,J_{1.2}10.8Hz,H-1), 5.13-5.19(m,1H,H-3 of C₁₄-O-C₂₄)

### Example 28

### N-[2-0-{2-Acetamido-2,3-dideoxy-6-0-octadecanoyl-1-S-((3R)-3-tetracosanoyloxytetradecanoyl)-1-thio-β-D-glucopyranos-3-yl}-D-lactoyl]-L-alanyl-D-isoglutamine methylester

The compound of the formula (I′) wherien R₁ is 3-tetracosanoyloxytetradecanoylthio (411.8mg, 0.380mmol) was dissolved in a mixture of dry dioxane (4.0ml) and dry DMF (1.5ml). To the solution were added octadeconoic acid (140.5mg, 0.494mmol), DCC(156.8mg, 0.760mmol) and DMAP (23.2mg, 0.190mmol), and the resultant was stirred for 3 hours at room temperature. In the same manner as that in Example 14, the title compound (223.5mg, yield:43.5%) was obtained.
mp : 147.5-149.0°C
[α]$\frac{\text{25}}{\text{D}}$: +17.40° ( C=0.632,CH₂Cl₂ MeOH=1 : 1)
IR γmax(cm⁻¹) : 3600-3200(OH), 3300(NH), 2950, 2880(CH), 1750(ester), 1660, 1560(amido)
NMR(CDCl₃) : 0.88(t,9H,JMeCH5.5Hz,3MeCH₂), 1.25(m,86H,43CH₂), 1.35(d,3H,JMeCH6.3Hz,MeC of Ala), 1.57(m,6H,3MeCH₂), 1.90(s,3H,AcN), 2.05-2.90(m,2H,CH₂CH of Gln), 2.35(t,2H,JCH₂CH₂7.8Hz,CH₂CO of Gln), 3.68(s,3H,COOMe), 5.13(d,1H,J_{1.2}11.0Hz,H-1), 5.15(m,1H,H-3 of C₁₄OC₂₄)
Pharmacological activities of the compounds of the present invention are shown as follows.

### (1) Hepatitis-vaccine enhancing activity (adjuvant activity)

A compound of the present invneiton was dissolved in lipidmicrosphere (1mg/ml). On the other hand, a solution of hepatitis B virus surface antigen (HBs) in physiological saline was prepared (50»g/ml). The above solutions in equal amounts were mixed to prepare a test solution. A control solution was prepared by the exclusion of the compound of the present invention from said test solution. A mixture of a suspension of aluminium hydroxide gel in physiological saline (1mg/ml) and said hepatitis vaccine preparation in equal amounts was prepared as another control solution. The test solution (0.2ml) was intraperitoneally administered to each mouse in one group consisting of seven female CDF₁ mice.

Blood samples were collected from the fundus ocluli vein of each mouse every week after the administration and then centrifuged to obtain serums. Three weeks after the administration, 0.2ml of the test liquid was intraperitoneally administered again to each mouse for secondary stimulation. Then blood collecting was conducted every week to obtain serums after the application of the secondary stimulation, in the same manner as that described above.

The amount of the IgG antibodies against the hepatitis B virus surface antigens (HBs) in the serums thus obtained was determined with an ELISA method. The results are shown in Table 1.

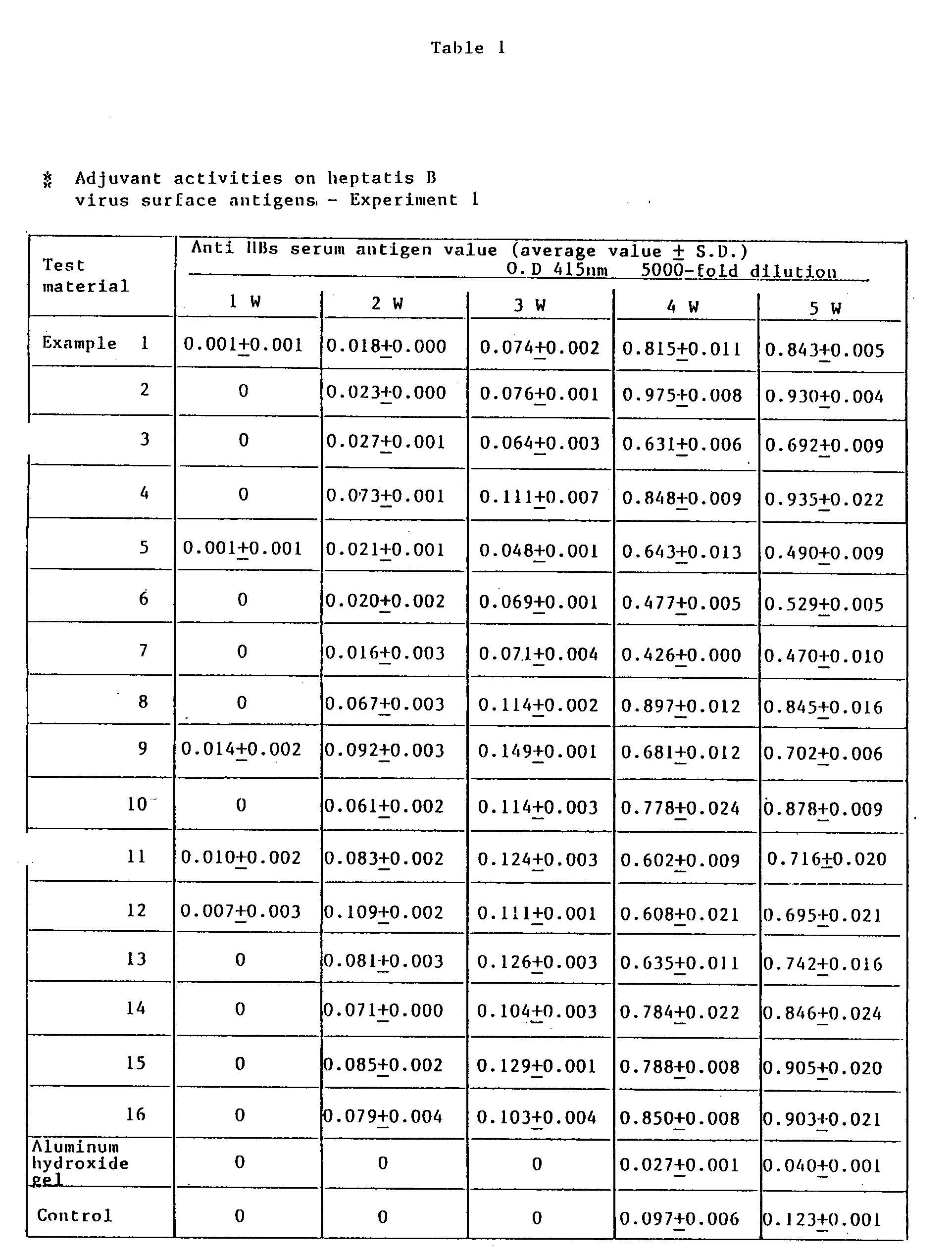

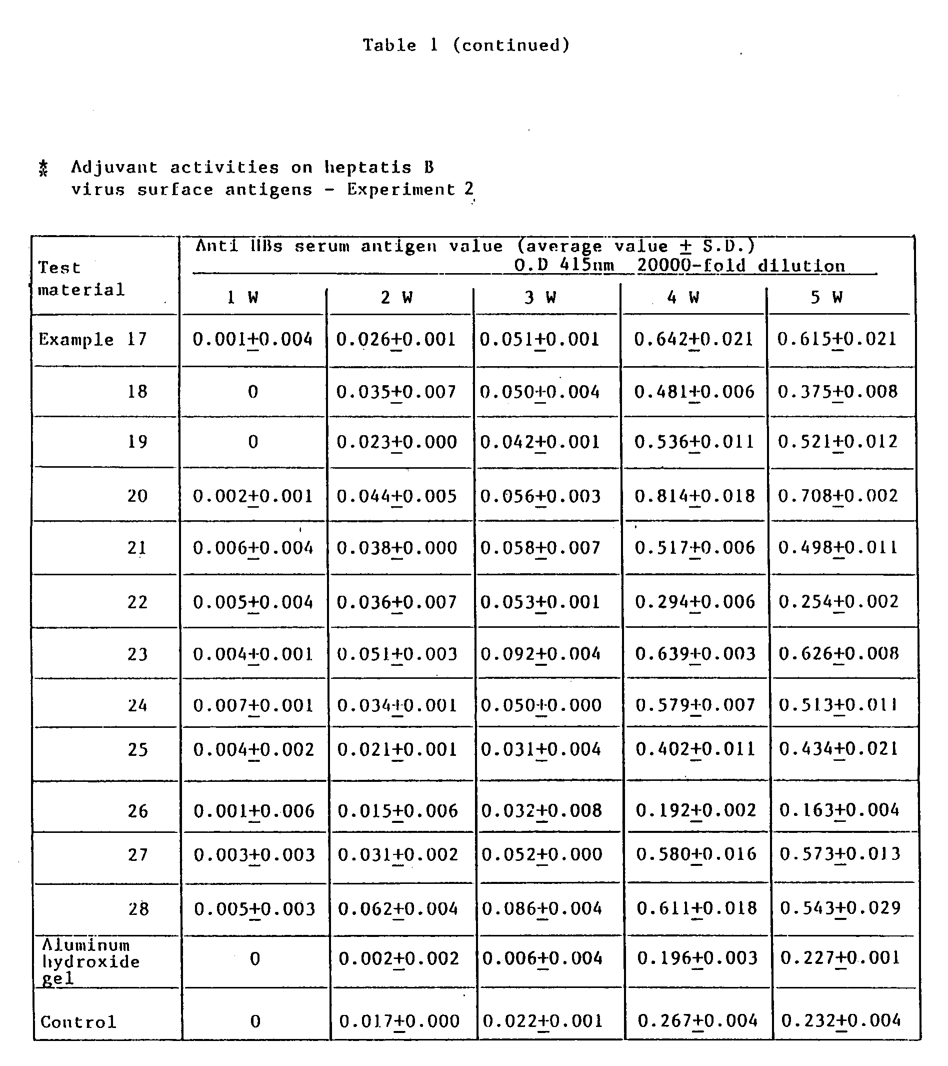

### (2) Influenza HA vaccine enhancing activity (adjuvant activity)

A compound of the present invention was dissolved in lipidmicrosphere (1mg/ml). On the other hand, a solution of influenza HA vaccine (B/nagasaki/1/87 strain) in physiological saline was prepared (100 ccA/ml). The above solutions in equal amounts were mixed to prepare a test solution. A control solution was made by the exclusion of the compound of the present inveniton from said test liquid. A mixture of a suspension of aluminium hydroxide gel in physiological saline(1mg/ml) and said influenza HA vaccine preparation in equal amounts was prepared as another control liquid. The test solution (0.2ml) was intraperitoneally administered to each mouse in one group consisting of seven female CDF₁ mice.

Blood samples were collected from the fundus oculi vein of each mouse every week after the administration and then centrifuged to obtain serums. Three weeks after the aministration, 0.2ml of the test liquid was intraperitoneally administered again to each mouse for secondary stimulation. Then, blood collecting was conducted every week to obtain serums after the application of the secondary stimulation, in the same manner as that described above.

The amount of the IgG antibodies against the influenza HA vaccine (B/Nagasaki/1/87 strain) in the serums thus obtained was determined with an ELISA mehtod. The results are shown in Table 2.

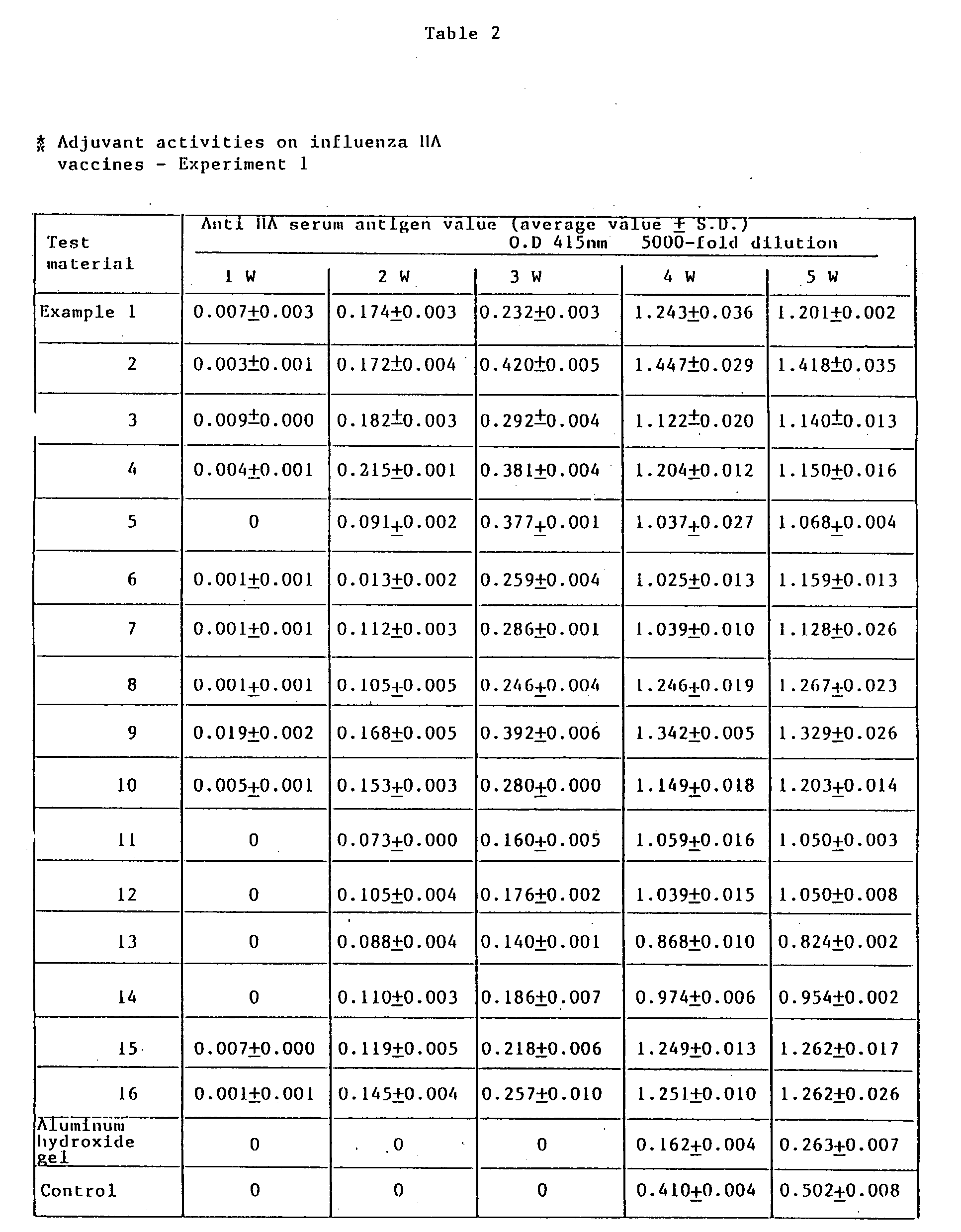

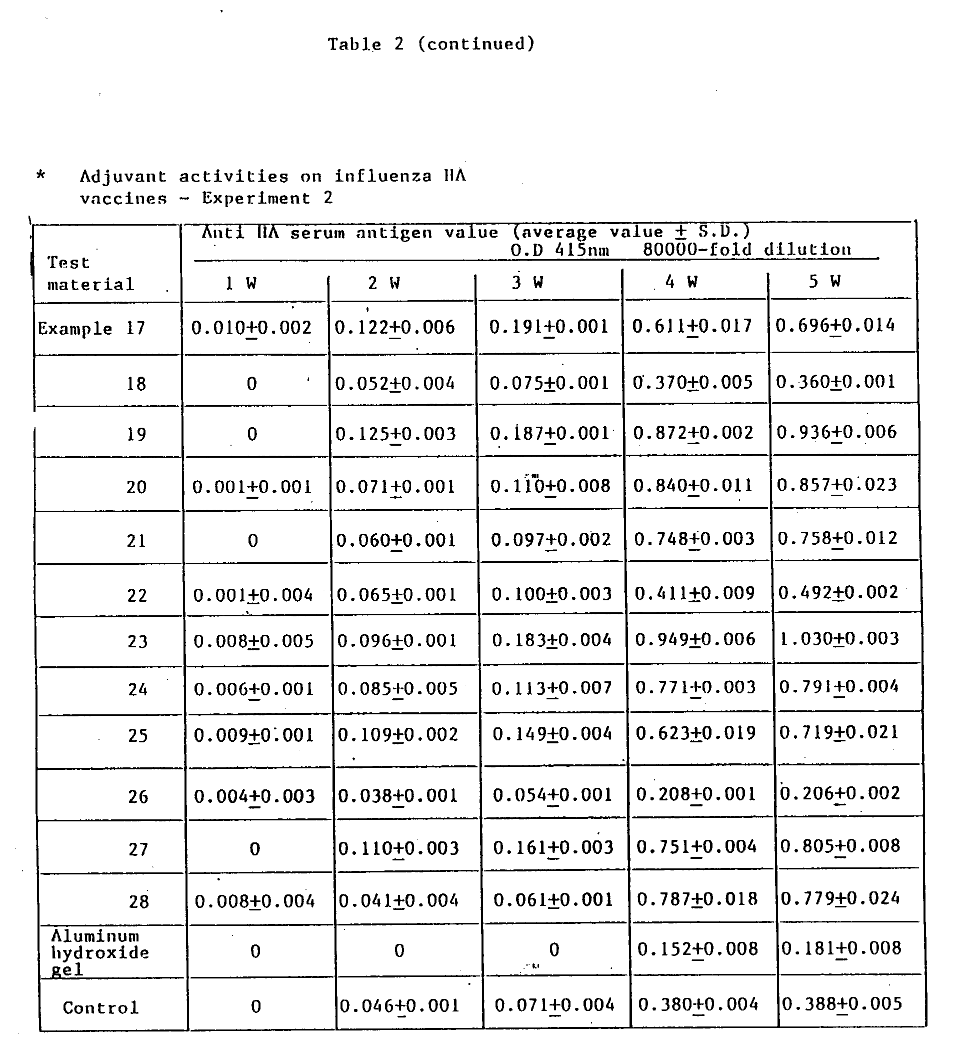

### (3) Activation of macrophages (an effect which inhibits the growth of tumor cells)

A compound of the present invention was dissolved in lipidmicrosphere to obtain a solution in a concentration of 500»g/ml, and 0.2ml of such solution was intraperitoneally administered to each mouse in one group consisting of seven female CDF₁ mice. Intraperitoneal macrophages obtained three days after the administration and L-1210 mouse leukemia cells were mixed in the ratio of cell numbers of 20 : 1, respectively. Two hundred »l of the mixture was placed in each well of one sheet of 96 well microtiter plate. After 72 hours, the increase of the cell number in each well was determined by a MTT assay method. The ratio of the cell number for the mixture of the L-1210 cells and the macrophage relative to the cell number for the L-1210 cells only(growth inhibitory ratio) was determined, and the results are shown in Table 3.

**Table 3**

| Test material | | Growth inhibitory ratio of L-1210 mouse leukemia cells(%) |
|---|---|---|
| Example | 1 | 40.0 |
| | 2 | 41.3 |
| | 3 | 42.6 |
| | 4 | 45.2 |
| | 5 | 57.8 |
| | 6 | 63.0 |
| | 7 | 90.2 |
| | 8 | 57.9 |
| | 9 | 58.5 |
| | 10 | 76.6 |
| | 11 | 59.7 |
| | 12 | 79.0 |
| | 13 | 39.7 |
| | 14 | 96.4 |
| | 15 | 87.5 |
| | 16 | 76.3 |
| Control | | 9.1 |

## Claims

1. A muramyl dipeptide derivative of the following formula (I): 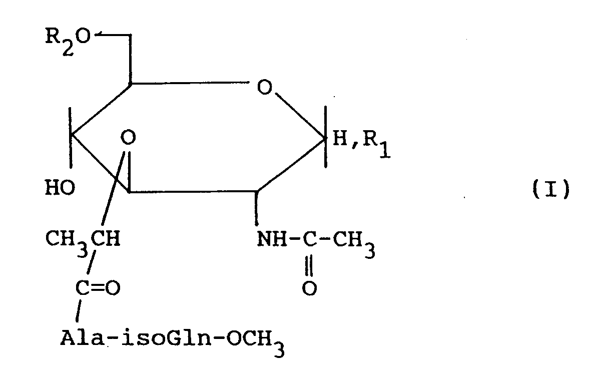 wherein "Ala" is 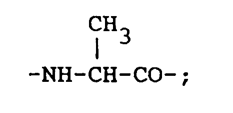 "isoGln" is 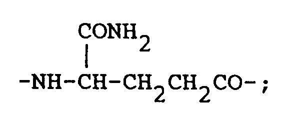 R¹ is R₃O- or R₃S-[R₃ is 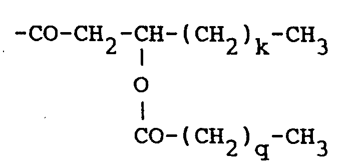 (k is an integer from 8 to 12; q is an integer from 10 to 22) or R₃ is 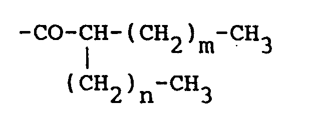 (m is an integer from 11 to 17; n is an integer from 11 to 17)]; and R₂ is hydrogen atom or -CO-(CH₂)ₚ-CH₃ (p is an integer from 8 to 22).

2. A compound of claim 1 wherein R₃ is 2-tetradecylhexadecanoyl.

3. A compound of claim 1 wherein R₃ is (3R)-3-tetradecanoyloxytetradecanoyl.

4. A compound of claim 1 wherein R₃ is (3R)-3-hexadecanoyloxytetradecanoyl.

5. A compound of claim 1 wherein R₂ is hydrogen atom.

6. A compound of claim 1 wherein R₂ is tetradecanoyl.

7. A compound of claim 1 wherein "Ala" is L-alanine residue, and "isoGln" is D-isoglutamine residue.

8. A compound of claim 1 which is
N-[2-0-{2-acetamido-2,3-dideoxy-1-0-(2-tetradecylhexadecanoyl)-α-D-glucopyranos-3-yl}-D-lactoyl]-L-alanyl-D-isoglutamine methylester,
N-[2-0-{2-acetamido-2,3-dideoxy-6-0-decanoyl-1-0-(2-tetradecylhexadecanoyl)-α-D-glucopyranos-3-yl}-D-lactoyl]-L-alanyl-D-isoglutamine methylester,
N-[2-0-{2-acetamido-2,3-dideoxy-6-0-tetradecanoyl-1-0-(2-tetradecylhexadecanoyl)-α-D-glucopyranos-3-yl}-D-lactoyl]-L-alanyl-D-isoglutamine methylester,
N-[2-0-{2-acetamido-2,3-dideoxy-6-0-octadecanoyl-1-0-(2-tetradecylhexadecanoyl)-α-D-glucopyranos-3-yl}-D-lactoyl]-L-alanyl-D-isoglutamine methylester,
N-[2-0-{2-acetamido-2,3-dideoxy-1-S-(2-tetradecylhexadecanoyl)-1-thio-β-D-glucopyranos-3-yl}-D-lactoyl]-L-alanyl-D-isoglutamine methylester,
N-[2-0-{2-acetamido-2,3-dideoxy-6-0-decanoyl-1-S-(2-tetradecylhexadecanoyl)-1-thio-β-D-glucopyranos-3-yl}-D-lactoyl]-L-alanyl-D-isoglutamine methylester,
N-[2-0-{2-acetamido-2,3-dideoxy-6-0-tetradecanoyl-1-S-(2-tetradecylhexadecanoyl)-1-thio-β-D-glucopyranos-3-yl}-D-lactoyl]-L-alanyl-D-isoglutamine methylester,
N-[2-0-{2-acetamido-2,3-dideoxy-6-0-octadecanoyl-1-S-(2-tetradecylhexadecanoyl)-1-thio-β-D-glucopyranos-3-yl}-D-lactoyl]-L-alanyl-D-isoglutamine methylester,
N-[2-0-{2-acetamido-2,3-dideoxy-1-0-((3R)-3-tetradecanoyloxytetradecanoyl)-α-D-glucopyranos-3-yl}-D-lactoyl]-L-alanyl-D-isoglutamine methylester,
N-[2-0-{2-acetamido-2,3-dideoxy-6-0-decanoyl-1-0-((3R)-3-tetradecanolyloxytetradecanoyl)-α-D-glucopyranos-3-yl}-D-lactoyl]-L-alanyl-D-isoglutamine methylester,
N-[2-0-{2-acetamido-2,3-dideoxy-6-0-tetradecanoyl-1-0-((3R)-3-tetradecanoyloxytetradecanoyl)-α-D-gluco pyranos-3-yl}-D-lactoyl]-L-alanyl-D-isoglutamine methylester,
N-[2-0-{2-acetamido-2,3-dideoxy-6-0-octadecanoyl-1-0-((3R)-3-tetradecanoyloxytetradecanoyl)-α-D-glucopyranos-3-yl}-D-lactoyl]-L-alanyl-D-isoglutamine methylester,
N-[2-0-{2-acetamido-2,3-dideoxy-1-S-((3R)-3-tetradecanoyloxytetradecanoyl)-1-thio-β-D-glucopyranos-3-yl}-D-lactoyl]-L-alanyl-D-isoglutamine methylester,
N-[2-0-{2-acetamido-2,3-dideoxy-6-0-decanoyl-1-S-((3R)-3-tetradecanoyloxytetradecanoyl)-1-thio-β-D-glucopyranos-3-yl}-D-lactoyl]-L-alanyl-D-isoglutamine methylester,
N-[2-0-{2-acetamido-2,3-dideoxy-6-0-tetradecanoyl-1-S-((3R)-3-tetradecanoyloxytetradecanoyl)-1-thio-β-D-glucopyranos-3-yl}-D-lactoyl]-L-alanyl-D-isoglutamine methylester,
N-[2-0-{2-acetamido-2,3-dideoxy-6-0-octadecanoyl-1-S-((3R)-3-tetradecanoyloxytetradecanoyl)-1-thio-β-D-glucopyranos-3-yl}-D-lactoyl]-L-alanyl-D-isoglutamine methylester,
N-[2-0-{2-acetamido-2,3-dideoxy-1-0-((3R)-3-hexadecanoyloxytetradecanoyl)-α-D-glucopyranos-3-yl}-D-lactoyl]-L-alanyl-D-isoglutamine methylester,
N-[2-0-{2-acetamido-2,3-dideoxy-1-0-((3R)-3-hexadecanoyloxytetradecanoyl)-6-0-octadecanoyl-α-D-glucopyranos-3-yl}-D-lactoyl]-L-alanyl-D-isoglutamine methylester,
N-[2-0-{2-acetamido-2,3-dideoxy-1-S-((3R)-3-hexadecanoyloxytetradecanoyl)-1-thio-β-D-glucopyranos-3-yl}-D-lactoyl]-L-alanyl-D-isoglutamine methylester,
N-[2-0-{2-acetamido-2,3-dideoxy-1-S-((3R)-3-hexadecanoyloxytetradecanoyl)-6-0-octadecanoyl-1-thio-β-D-glucopyranos-3-yl}-D-lactoyl]-L-alanyl-D-isoglutamine methylester,
N-[2-0-{2-acetamido-2,3-dideoxy-1-0-((3R)-3-octadecanoyloxytetradecanoyl)-α-D-glucopyranos-3-yl}-D-lactoyl]-L-alanyl-D-isoglutamine methylester,
N-[2-0-{2-acetamido-2,3-dideoxy-6-0-octadecanoyl-1-0-((3R)-3-octadecanoyloxytetradecanoyl)-α-D-glucopyranos-3-yl}-D-lactoyl]-L-alanyl-D-isoglutamine methylester,
N-[2-0-{2-acetamido-2,3-dideoxy-1-S-((3R)-3-octadecanoyloxytetradecanoyl)-1-thio-β-D-glucopyranos-3-yl}-D-lactoyl]-L-alanyl-D-isoglutmine methylester,
N-[2-0-{2-acetamido-2,3-dideoxy-6-0-octadecanoyl-1-S-((3R)-3-octadecanoyloxytetradecanoyl)-1-thio-β-D-glucopyranos-3-yl}-D-lactoyl]-L-alanyl-D-isoglutamine methylester,
N-[2-0-{2-acetamido-2,3-dideoxy-1-0-((3R)-3-tetracosanoyloxytetradecanoyl)-α-D-glucopyranos-3-yl}-D-lactoyl]-L-alanyl-D-isoglutamine methylester,
N-[2-0-{2-acetamido-2,3-dideoxy-6-0-octadecanoyl1-0-((3R)-3-tetracosanoyloxytetradecanoyl)-α-D-glucopyranos-3-yl}-D-lactoyl]-L-alanyl-D-isoglutamine methylester,
N-[2-0-{2-acetamido-2,3-dideoxy-1-S-((3R)-3-tetracosanoyloxytetradecanoyl)-1-thio-β-D-glucopyranos-3-yl}-D-lactoyl]-L-alanyl-D-isoglutamine methylester, or
N-[2-0-{2-acetamido-2,3-dideoxy-6-0-octadecanoyl-1-S-((3R)-3-tetracosanoyloxytetradecanoyl)-1-thio-β-D-glucopyranos-3-yl}-D-lactoyl]-L-alanyl-D-isoglutamine methylester.

9. An immunoregulating composition comprising a muramyl dipeptide derivative of the formula (I) as defined in claim 1 and a pharmaceutically acceptable carrier.

10. An immunoregulating composition of claim 9 in which the muramyl dipeptide derivative (I) is a compound in accordance with any one of claims 2 to 8.

11. An immunoregulating composition of claim 9 which is used for enhancing in vivo activity of a BCG, hepatitis or influenza virus vaccines.

12. An immunoregulating composition of claim 9 which is used for enhancing in vivo activity of antibacterial agents.

13. An immunoregulating composition of claim 9 which is used for enhancing in vivo activity of antitumor agents.

## Patentansprüche

1. Muramyldipeptidderivat der Formel (I): 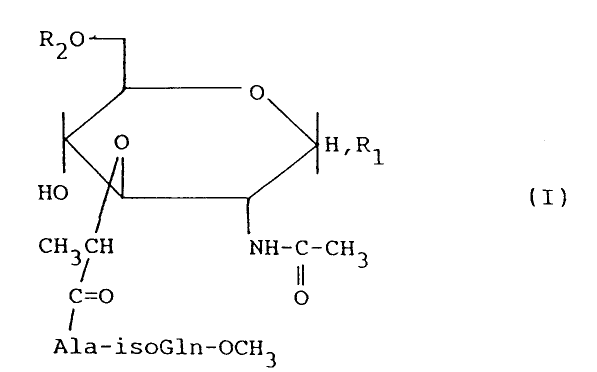 worin "Ala" für 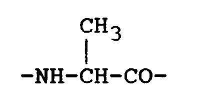 steht; "isoGln" für 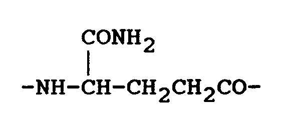 steht; R¹ für R₃O- oder R₃S- steht [R₃ ist 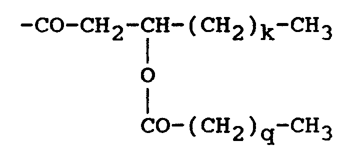 (k ist eine ganze Zahl von 8 bis 12; q ist eine ganze Zahl von 10 bis 22) oder R₃ ist 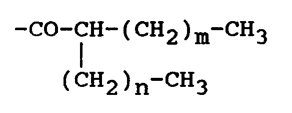 (m ist eine ganze Zahl von 11 bis 17; n ist eine ganze Zahl von 11 bis 17)]; und R₂ für ein Wasserstoffatom oder -CO-(CH₂)ₚ-CH₃ steht (p ist eine ganze Zahl von 8 bis 22).

2. Verbindung nach Anspruch 1, dadurch **gekennzeichnet,** daß R₃ für 2-Tetradecylhexadecanoyl steht.

3. Verbindung nach Anspruch 1, dadurch **gekennzeichnet,** daß R₃ für (3R)-3-Tetradecanoyloxytetradecanoyl steht.

4. Verbindung nach Anspruch 1, dadurch **gekennzeichnet,** daß R₃ für (3R)-3-Hexadecanoyloxytetradecanoyl steht.

5. Verbindung nach Anspruch 1, dadurch **gekennzeichnet,** daß R₂ für ein Wasserstoffatom steht.

6. Verbindung nach Anspruch 1, dadurch **gekennzeichnet,** daß R₂ für Tetradecanoyl steht.

7. Verbindung nach Anspruch 1, dadurch **gekennzeichnet,** daß "Ala" der L-Alaninrest ist und daß "isoGln" der D-Isoglutaminrest ist.

8. Verbindung nach Anspruch 1, nämlich
N-[2-O-{2-Acetamido-2,3-didesoxy-1-O-(2-tetradecylhexadecanoyl)-α-D-glucopyranos-3-yl}-D-lactoyl]-L-alanyl-D-isoglutaminmethylester,
N-[2-O-{2-Acetamido-2,3-didesoxy-6-O-decanoyl-1-O-(2-tetradecylhexadecanoyl)-α-D-glucopyranos-3-yl}-D-lactoyl]-L-alanyl-D-isoglutaminmethylester,
N-[2-O-{2-Acetamido-2,3-didesoxy-6-O-tetradecanoyl-1-O-(2-tetradecylhexadecanoyl)-α-D-glucopyranos-3-yl}-D-lactoyl]-L-alanyl-D-isoglutaminmethylester,
N-[2-O-{2-Acetamido-2,3-didesoxy-6-O-octadecanoyl-1-O-(2-tetradecylhexadecanoyl)-α-D-glucopyranos-3-yl}-D-lactoyl]-L-alanyl-D-isoglutaminmethylester,
N-[2-O-{2-Acetamido-2,3-didesoxy-1-S-(2-tetradecylhexadecanoyl)-1-thio-β-D-glucopyranos-3-yl}-D-lactoyl]-L-alanylD-isoglutaminmethylester,
N-[2-O-{2-Acetamido-2,3-didesoxy-6-O-decanoyl-1-S-(2-tetradecylhexadecanoyl)-1-thio-β-D-glucopyranos-3-yl}-D-lactoyl]-L-alanyl-D-isoglutaminmethylester,
N-[2-O-{2-Acetamido-2,3-didesoxy-6-O-tetradecanoyl-1-S-(2-tetradecylhexadecanoyl)-1-thio-β-D-glucopyranos-3-yl}-D-lactoyl]-L-alanyl-D-isoglutaminmethylester,
N-[2-O-{2-Acetamido-2,3-didesoxy-6-O-octadecanoyl-1-S-(2-tetradecylhexadecanoyl)-1-thio-β-D-glucopyranos-3-yl}-D-lactoyl]-L-alanyl-D-isoglutaminmethylester,
N-[2-O-{2-Acetamido-2,3-didesoxy-1-O-((3R)-3-tetradecanoyloxytetradecanoyl)-α-D-glucopyranos-3-yl}-D-lactoyl)-L-alanyl-D-isoglutaminmethylester,
N-[2-O-{2-Acetamido-2,3-didesoxy-6-O-decanoyl-1-O-((3R)-3-tetradecanoyloxytetradecanoyl)-α-D-glucopyranos-3-yl}-D-lactoyl]-L-alanyl-D-isoglutaminmethylester,
N-[2-O-{2-Acetamido-2,3-didesoxy-6-O-tetradecanoyl-1-O-((3R)-3-tetradecanoyloxytetradecanoyl)-α-D-glucopyranos-3-yl}-D-lactoyl]-L-alanyl-D-isoglutaminmethylester,
N-[2-O-{2-Acetamido-2,3-didesoxy-6-O-octadecanoyl-1-O-((3R)-3-tetradecanoyloxytetradecanoyl)-α-D-glucopyranos-3-yl}-D-lactoyl]-L-alanyl-D-isoglutaminmethylester,
N-[2-O-{2-Acetamido-2,3-didesoxy-1-S-((3R)-3-tetradecanoyloxytetradecanoyl)-1-thio-β-D-glucopyranos-3-yl}-D-lactoyl]-L-alanyl-D-isoglutaminmethylester,
N-[2-O-{2-Acetamido-2,3-didesoxy-6-O-decanoyl-1-S-((3R)-3-tetradecanoyloxytetradecanoyl)-1-thio-β-D-glucopyranos-3-yl}-D-lactoyl]-L-alanyl-D-isoglutaminmethylester,
N-{2-O-{2-Acetamido-2,3-didesoxy-6-O-tetradecanoyl-1-S-((3R)-3-tetradecanoyloxytetradecanoyl)-1-thio-β-D-glucopyranos-3-yl}-D-lactoyl]-L-alanyl-D-isoglutaminmethylester,
N-[2-O-{2-Acetamido-2,3-didesoxy-6-O-octadecanoyl-1-S-((3R)-3-tetradecanoyloxytetradecanoyl)-1-thio-β-D-glucopyranos-3-yl}-D-lactoyl]-L-alanyl-D-isoglutaminmethylester,
N-[2-O-{2-Acetamido-2,3-didesoxy-1-O-((3R)-3-hexadecanoyloxytetradecanoyl)-α-D-glucopyranos-3-yl}-D-lactoyl]-L-alanyl-D-isoglutaminmethylester,
N-[2-O-{2-Acetamido-2,3-didesoxy-1-O-((3R)-3-hexadecanoyloxytetradecanoyl)-6-O-octadecanoyl-α-D-glucopyranos-3-yl}-D-lactoyl]-L-alanyl-D-isoglutaminmethylester,
N-[2-O-{2-Acetamido-2,3-didesoxy-1-S-((3R)-3-hexadecanoyloxytetradecanoyl)-1-thio-β-D-glucopyranos-3-yl}-D-lactoyl]-L-alanyl-D-isoglutaminmethylester,
N-[2-O-{2-Acetamido-2,3-didesoxy-1-S-((3R)-3-hexadecanoyloxytetradecanoyl)-6-O-octadecanoyl-1-thio-β-D-glucopyranos-3-yl}-D-lactoyl]-L-alanyl-D-isoglutaminmethylester,
N-[2-O-{2-Acetamido-2,3-didesoxy-1-O-((3R)-3-octadecanoyloxytetradecanoyl)-α-D-glucopyranos-3-yl}-D-lactoyl]-L-alanyl-D-isoglutaminmethylester,
N-[2-O-{2-Acetamido-2,3-didesoxy-6-O-octadecanoyl-1-O-((3R)-3-octadecanoyloxytetradecanoyl)-α-D-glucopyranos-3-yl}-D-lactoyl]-L-alanyl-D-isoglutaminmethylester,
N-[2-O-{2-Acetamido-2,3-didesoxy-1-S-((3R)-3-octadecanoyloxytetradecanoyl)-1-thio-β-D-glucopyranos-3-yl}-D-lactoyl]-L-alanyl-D-isoglutaminmethylester,
N-[2-O-{2-Acetamido-2,3-didesoxy-6-O-octadecanoyl-1-S-((3R)-3-octadecanoyloxytetradecanoyl)-1-thio-β-D-glucopyranos-3-yl}-D-lactoyl]-L-alanyl-D-isoglutaminmethylester,
N-[2-O-{2-Acetamido-2,3-didesoxy-1-O-((3R)-3-tetracosanoyloxytetradecanoyl)-α-D-glucopyranos-3-yl}-D-lactoyl]-L-alanyl-D-isoglutaminmethylester,
N-[2-O-{2-Acetamido-2,3-didesoxy-6-O-octadecanoyl-1-O-((3R)-3-tetracosanoyloxytetradecanoyl)-α-D-glucopyranos-3-yl}-D-lactoyl]-L-alanyl-D-isoglutaminmethylester,
N-[2-O-{2-Acetamido-2,3-didesoxy-1-S-((3R)-3-tetracosanoyloxytetradecanoyl)-1-thio-β-D-glucopyranos-3-yl}-D-lactoyl]-L-alanyl-D-isoglutaminmethylester oder
N-[2-O-{2-Acetamido-2,3-didesoxy-6-O-octadecanoyl-1-S-((3R)-3-tetracosanoyloxytetradecanoyl)-1-thio-β-D-glucopyranos-3-yl}-D-lactoyl]-L-alanyl-D-isoglutaminmethylester.

9. Immunregulierendes Präparat, dadurch **gekennzeichnet,** daß es ein Muramyldipeptidderivat der Formel (I) nach Anspruch 1 und einen pharmazeutisch annehmbaren Träger enthält.

10. Immunregulierendes Präparat nach Anspruch 9, dadurch **gekennzeichnet,** daß das Muramyldipeptidderivat (I) eine Verbindung nach einem der Ansprüche 2 bis 8 ist.

11. Immunregulierendes Präparat nach Anspruch 9, dadurch **gekennzeichnet,** daß es zur Verstärkung der invivo-Aktivität von BCG-, Hepatitis- oder Influenzavirus-Impfstoffen verwendet wird.

12. Immunregulierendes Präparat nach Anspruch 9, dadurch **gekennzeichnet,** daß es zur Verstärkung der invivo-Aktivität von antibakteriellen Mitteln verwendet wird.

13. Immunregulierendes Präparat nach Anspruch 9, dadurch **gekennzeichnet,** daß es zur Verstärkung der invivo-Aktivität von Anti-Tumormitteln verwendet wird.

## Revendications

1. Dérivé de muramyle dipeptide représenté par la formule suivante : 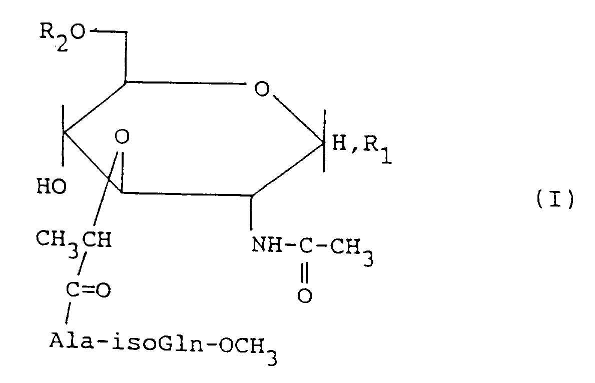 dans laquelle "Ala" est 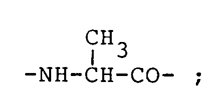 "isoGln" est 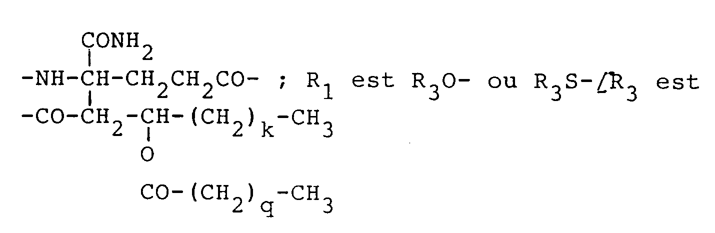 (k est un entier de 8 à 12 ; q est un entier de 10 à 22) ou R₃ est 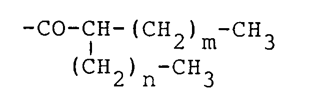 (m est un entier de 11 à 17 ; n est un entier de 1 à 17)] ; et R₂ est un atome d'hydrogène ou un groupe -CO-(CH₂)ₚ-CH₃ (p est un entier de 8 à 22).

2. Composé suivant la revendication 1, dans lequel R₃ est un groupe 2-tétradécylhexadécanoyle.

3. Composé suivant la revendication 1, dans lequel R₃ est un groupe (3R)-3-tétradécanoyloxytétradécanoyle.

4. Composé suivant la revendication 1, dans lequel R₃ est un groupe (3R)-3-hexadécanoyloxytétradécanoyle.

5. Composé suivant la revendication 1, dans lequel R₂ est un atome d'hydrogène.

6. Composé suivant la revendication 1, dans lequel R₂ est un groupe tétradécanoyle.

7. Composé suivant la revendication 1, dans lequel "Ala" est un résidu de L-alanine, et "isoGln" est un résidu de D-isoglutamine.

8. Composé suivant la revendication 1 qui est l'ester méthylique de N-[2-0-{2-acétamido-2,3-didésoxy-1-O-(2-tétradécylhexadécanoyle)-α-D-glucopyranos-3-yl}-D-lactoyl]-L-alanyl-D-isoglutamine,
l'ester méthylique de N-[2-0-{2-acétamido-2,3-didésoxy6-0-décanoyl-1-0-(2-tétradécylhexadécanoyl)-α-D-glucopyranos-3-yl}-D-lactoyl]-L-alanyl-D-isoglutamine,
l'ester méthylique de N-[2-0-{2-acétamido-2,3-didésoxy-6-0-tétradécanoyl-1-0-(2-tétradécylhexadécanoyl)-α-D-glucopyranos-3-yl}-D-lactoyl]-L-alanyl-D-isoglutamine,
l'ester méthylique de N-/2-0-{2-acétamido-2,3-didésoxy-6-0-octadécanoyl-1-0-(2-tétradécylhexadécanoyl)-α-D-glucopyranos-3-yl}-D-lactoyl]-L-alanyl-D-isoglutamine,
l'ester méthylique de N-[2-0-{2-acétamido-2,3-didésoxy-1-S-(2-tétradécylhexadécanoyl)-1-thio-β-D-glucopyranos-3-yl}-D-lactoyl]-L-alanyl-D-isoglutamine,
l'ester méthylique de N-[2-0-{2-acétamido-2,3-didésoxy-6-0-décanoyl-1-S-(2-tétradécylhexadécanoyl)-1-thio-β-D-glucopyranos-3-yl}-D-lactoyl]-L-alanyl-D-isoglutamine,
l'ester méthylique de N-[2-0-{2-acétamido-2,3-didésoxy-6-0-tétradécanoyl-1-S-(2-tétradécylhexadécanoyl)-1-thio-β-D-glucopyranos-3-yl}-D-lactoyl]-L-alanyl-D-isoglutamine,
l'ester méthylique de N-[2-0-{2-acétamido-2,3-didésoxy-6-0-octadécanoyl-1-S-(2-tétradécylhexadécanoyl)-1-thio-β-D-glucopyranos-3-yl}-D-lactoyl]-L-alanyl-D-isoglutamine,
l'ester méthylique de N-[2-0- 2-acétamido-2,3-didésoxy-1-0-((3R)-3-tétradécanoyloxytétradécanoyl)-α-D-glucopyranos-3-yl}-D-lactoyl]-L-alanyl-D-isoglutamine,
l'ester méthylique de N-[2-0-{2-acétamido-2,3-didésoxy-6-0-décanoyl-1-0-((3R)-3-tétradécanoyloxytétradécanoyl)-α-D-glucopyranos-3-yl}-D-lactoyl]-L-alanyl-D-isoglutamine,
l'ester méthylique de N-[2-0-{2-acétamido-2,3-didésoxy-6-0-tétradécanoyl-1-0-((3R)-3-tétradécanoyloxytétradéca-noyl)-α-D-glucopyranos-3-yl}-D-lactoyl]-L-alanyl-D-iso-glutamine,
l'ester méthylique de N-[2-0-{2-acétamido-2,3-didésoxy-6-0-octadécanoyl-1-0-((3R)-3-tétradécanoyloxytétradécanoyl)-α-D-glucopyranos-3-yl}-D-lactoyl]-L-alanyl-D-isoglutamine,
l'ester émthylique de N-[2-0-{2-acétamido-2,3-didésoxy-1-S-((3R)-3-tétradécanoyloxytétradécanoyl)-1-thio-β-D-glucopyranos-3-yl}-D-lactoyl]-L-alanyl-D-isoglutamine,
l'ester méthylique de N-[2-0-{2-acétamido-2,3-didésoxy-6-0-décanoyl-1-S-((3R)-3-tétradécanoyloxytétradécanoyl)-1-thio-β-D-glucopyranos-3-yl}-D-lactoyl]-L-alanyl-D-isoglutamine,
l'ester méthylique de N-[2-0-{2-acétamido-2,3-didésoxy-6-0-tétradécanoyl-1-S-((3R)-3-tétradécanoyloxytétradéca-noyl)-1-thio-β-D-glucopyranos-3-yl}-D-lactoyl]-L-alanyl-D-isoglutamine,
l'ester méthylique de N-[2-0-{2-acétamido-2,3-didésoxy-6-0-octanedécanoyl-1-S-((3R)-3-tétradécanoyloxytétradécanoyl)-1-thio-β-D-glucopyranos-3-yl}-D-lactoyl]-L-alanyl-D-isoglutamine,
l'ester méthylique de N-[2-0-{2-acétamido-2,3-didésoxy-1-0-((3R)-3-hexadécanoyloxytétradécanoyl)-α-D-glucopyranos-3-yl}-D-lactoyl]-L-alanyl-D-isoglutamine,
l'ester méthylique de N-[2-0-{2-acétamido-2,3-didésoxy-1-0-((3R)-3-hexadécanoyloxytétradécanoyl)-6-0-octadécanoyl-α-D-glucopyranos-3-yl}-D-lactoyl]-L-alanyl-D-iso-glutamine,
l'ester méthylique de N-[2-0-{2-acétamido-2,3-didésoxy-1-S-((3R)-3-hexadécanoyloxytétradécanoyl)-1-thio-β-D-glucopyranos-3-yl}-D-lactoyl]-L-alanyl-D-isoglutamine,
l'ester méthylique de N-[2-0-{2-acétamido-2,3-didésoxy-1-0-((3R)-3-octadécanoyloxytétradécanoyl)-α-D-glucopyranos-3-yl}-D-lactoyl]-L-alanyl-D-isoglutamine,
l'ester méthylique de N-[2-0-{2-acétamido-2,3-didésoxy-6,0-octédécanoyl-1-0-((3R)-3-octadécanoyloxytétradécanoyl)-α-D-glucopyranos-3-yl}-D-lactoyl]-L-alanyl-D-isoglutamine,
l'ester méthylique de N-[2-0-{2-acétamido-2,3-didésoxy-1-S-((3R)-3-octadécanoyloxytétradécanoyl)-1-thio-β-D-glucopyranos-3-yl}-D-lactoyl]-L-alanyl-D-isoglutamine,
l'ester méthylique de N-[2-0-{2-acétamido-2,3-didésoxy-6-0-octadécanoyl-1-S-((3R)-3-octadécanoyloxytétradécanoyl)-1-thio-β-D-glucopyranos-3-yl}-D-lactoyl]-L-alanyl-D-isoglutamine,
l'ester méthylique de N-[2-0-{2-acétamido-2,3-didésoxy-1-0-((3R)-3-tétracosanoyloxytétradécanoyl)-α-D-glucopyranos-3-yl}-D-lactoyl]-L-alanyl-D-isoglutamine,
l'ester méthylique de N-[2-0-{2-acétamido-2,3-didésoxy-6-0-octadécanoyl-1-0-((3R)-3-tétracosanoyloxytétradécanoyl)-α-D-glucopyranos-3-yl}-D-lactoyl]-L-alanyl-D-isoglutamine,
l'ester méthylique de N-[2-0-{2-acétamido-2,3-didésoxy-1-S-((3R)-3-tétracosanoyloxytétradécanoyl)-1-thio-β-D-glucopyranos-3-yl}-D-lactoyl]-L-alanyl-D-isoglutamine, ou
l'ester méthylique de N-[2-0-{2-acétamido-2,3-didésoxy-6-0-octadécanoyl-1-S-((3R)-3-tétracosanoyloxytétradécanoyl)-1-thio-β-D-glucopyranos-3-yl}-D-lactoyl]-L-alanyl-D-isoglutamine.

9. Composition immunorégulatrice comprenant un dérivé de muramyle dipeptide de formule (I) suivant la revendication 1 et un support acceptable du point de vue pharmaceutique.

10. Composition immunorégulatrice suivant la revendication 9, dans laquelle le dérivé de muramyle dipeptide (I) est un composé suivant l'une quelconque des revendications 2 à 8.

11. Composition immunorégulatrice suivant la revendication 9, qui est utilisée pour augmenter l'activité in vivo d'un BCG, et de vaccins contre le virus de l'hépatite ou de l'influenza.

12. Composition immunorégulatrice suivant la revendication 9, qui est utilisée pour augmenter l'activité in vivo d'agents antibactériens.

13. Composition immunorégulatrice suivant la revendication 9, qui est utilisée pour augmenter l'activité in vivo d'agents antitumoraux.
